# EUROPEAN PATENT APPLICATION

(11) **EP 1 717 224 A1**
(43) Date of publication of application: **02.11.2006**
(21) Application number: 05709667.9
(22) Date of filing: 03.02.2005
(51) Int. Cl.: C07C 279/14, A61K 31/198, A61K 45/00, A61P 19/02, A61P 29/00, A61P 43/00

(54) **PEPTIDYL ARGININE DEIMINASE TYPE IV INHIBITOR**

(30) Priority: 04.02.2004 JP 2004028467
(71) Applicant: Yokohama City University, Yokohama-shi Kanagawa 236-0027 (JP)
(72) Inventor: SATO, Mamoru, Miura-shi, Kanagawa 238-0111 (JP); SHIMIZU, Toshiyuki, Yokohama-shi, Kanagawa 236-0017 (JP); HASHIMOTO, Hiroshi, Yokohama-shi, Kanagawa 230-0062 (JP); YAMADA, Michiyuki, Yokohama-shi, Kanagawa 236-0045 (JP); HIDAKA, Yuji, Ibaragi-shi, Osaka 567-0023 (JP)
(74) Representative: Woods, Geoffrey Corlett
(86) International application number: PCT/JP2005/001574
(87) International publication number: WO 2005/075414

(57) **Abstract**

A compound represented by the general formula (I) or a salt thereof is provided: 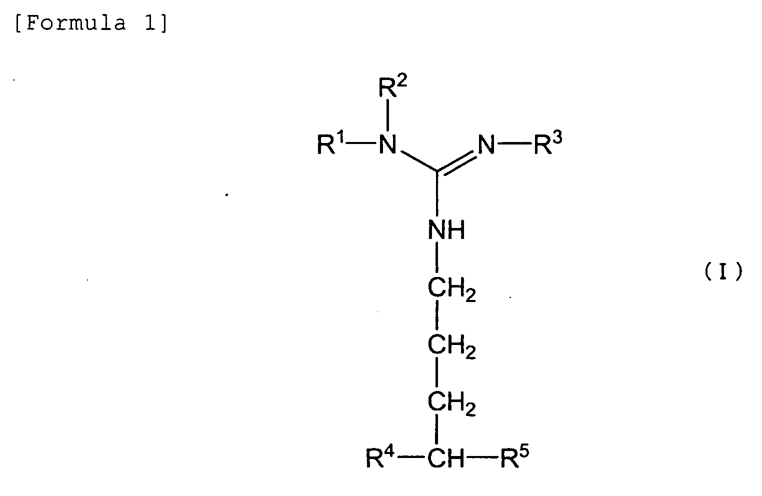 wherein R¹, R² and R³ each independently represent a hydrogen atom or an alkyl group having 1 to 3 carbon atoms, provided that at least one of R¹, R² and R³ does not represent a hydrogen atom; R⁴ represents an amino group which has a substituent; and R⁵ represents a carboxyl group which may have a substituent. Also provided is a peptidylarginine deiminase 4 inhibitor. The inhibitor can be used for the prevention and/or treatment of diseases associated with peptidylarginine deiminases (e.g., rheumatoid arthritis and multiple sclerosis).

SEQUENCE LISTING

<110> City of Yokohama

<120> Peptidylarginine Deiminase 4 inhibitors

<130> FP-036PCT

<140>
<141>

<150> JP P2004-028467
<151> 2004-02-04

<160> 1

<170> PatentIn Ver. 2.1

<210> 1
<211> 663
<212> PRT
<213> Homo sapiens

<400> 1
Met Ala Gln Gly Thr Leu Ile Arg Val Thr Pro Glu Gln Pro Thr His
  1               5                  10                  15

Ala Val Cys Val Leu Gly Thr Leu Thr Gln Leu Asp Ile Cys Ser Ser
             20                  25                  30

Ala Pro Glu Asp Cys Thr Ser Phe Ser Ile Asn Ala Ser Pro Gly Val
         35                  40                  45

Val Val Asp Ile Ala His Ser Pro Pro Ala Lys Lys Lys Ser Thr Gly
     50                  55                  60

Ser Ser Thr Trp Pro Leu Asp Pro Gly Val Glu Val Thr Leu Thr Met
 65                  70                  75                  80

Lys Ala Ala Ser Gly Ser Thr Gly Asp Gln Lys Val Gln Ile Ser Tyr
                 85                  90                  95

Tyr Gly Pro Lys Thr Pro Pro Val Lys Ala Leu Leu Tyr Leu Thr Ala
            100                 105                 110

Val Glu Ile Ser Leu Cys Ala Asp Ile Thr Arg Thr Gly Lys Val Lys
        115                 120                 125

Pro Thr Arg Ala Val Lys Asp Gln Arg Thr Trp Thr Trp Gly Pro Cys
    130                 135                 140

Gly Gln Gly Ala Ile Leu Leu Val Asn Cys Asp Arg Asp Asn Leu Glu
145                 150                 155                 160

Ser Ser Ala Met Asp Cys Glu Asp Asp Glu Val Leu Asp Ser Glu Asp
                165                 170                 175

Leu Gln Asp Met Ser Leu Met Thr Leu Ser Thr Lys Thr Pro Lys Asp
            180                 185                 190

Phe Phe Thr Asn His Thr Leu Val Leu His Val Ala Arg Ser Glu Met
        195                 200                 205

Asp Lys Val Arg Val Phe Gln Ala Thr Arg Gly Lys Leu Ser Ser Lys
    210                 215                 220

Cys Ser Val Val Leu Gly Pro Lys Trp Pro Ser His Tyr Leu Met Val
225                 230                 235                 240

Pro Gly Gly Lys His Asn Met Asp Phe Tyr Val Glu Ala Leu Ala Phe
                245                 250                 255

Pro Asp Thr Asp Phe Pro Gly Leu Ile Thr Leu Thr Ile Ser Leu Leu
            260                 265                 270

Asp Thr Ser Asn Leu Glu Leu Pro Glu Ala Val Val Phe Gln Asp Ser
        275                 280                 285

Val Val Phe Arg Val Ala Pro Trp Ile Met Thr Pro Asn Thr Gln Pro
    290                 295                 300

Pro Gln Glu Val Tyr Ala Cys Ser Ile Phe Glu Asn Glu Asp Phe Leu
305                 310                 315                 320

Lys Ser Val Thr Thr Leu Ala Met Lys Ala Lys Cys Lys Leu Thr Ile
                325                 330                 335

Cys Pro Glu Glu Glu Asn Met Asp Asp Gln Trp Met Gln Asp Glu Met
            340                 345                 350

Glu Ile Gly Tyr Ile Gln Ala Pro His Lys Thr Leu Pro Val Val Phe
        355                 360                 365

Asp Ser Pro Arg Asn Arg Gly Leu Lys Glu Phe Pro Ile Lys Arg Val
    370                 375                 380

Met Gly Pro Asp Phe Gly Tyr Val Thr Arg Gly Pro Gln Thr Gly Gly
385                 390                 395                 400

Ile Ser Gly Leu Asp Ser Phe Gly Asn Leu Glu Val Ser Pro Pro Val
                405                 410                 415

Thr Val Arg Gly Lys Glu Tyr Pro Leu Gly Arg Ile Leu Phe Gly Asp
            420                 425                 430

Ser Cys Tyr Pro Ser Asn Asp Ser Arg Gln Met His Gln Ala Leu Gln
        435                 440                 445

Asp Phe Leu Ser Ala Gln Gln Val Gln Ala Pro Val Lys Leu Tyr Ser
    450                 455                 460

Asp Trp Leu Ser Val Gly His Val Asp Glu Phe Leu Ser Phe Val Pro
465                 470                 475                 480

Ala Pro Asp Arg Lys Gly Phe Arg Leu Leu Leu Ala Ser Pro Arg Ser
                485                 490                 495

Cys Tyr Lys Leu Phe Gln Glu Gln Gln Asn Glu Gly His Gly Glu Ala
            500                 505                 510

Leu Leu Phe Glu Gly Ile Lys Lys Lys Lys Gln Gln Lys Ile Lys Asn
        515                 520                 525

Ile Leu Ser Asn Lys Thr Leu Arg Glu His Asn Ser Phe Val Glu Arg
    530                 535                 540

Cys Ile Asp Trp Asn Arg Glu Leu Leu Lys Arg Glu Leu Gly Leu Ala
545                 550                 555                 560

Glu Ser Asp Ile Ile Asp Ile Pro Gln Leu Phe Lys Leu Lys Glu Phe
                565                 570                 575

Ser Lys Ala Glu Ala Phe Phe Pro Asn Met Val Asn Met Leu Val Leu
            580                 585                 590

Gly Lys His Leu Gly Ile Pro Lys Pro Phe Gly Pro Val Ile Asn Gly
        595                 600                 605

Arg Cys Cys Leu Glu Glu Lys Val Cys Ser Leu Leu Glu Pro Leu Gly
    610                 615                 620

Leu Gln Cys Thr Phe Ile Asn Asp Phe Phe Thr Tyr His Ile Arg His
625                 630                 635                 640

Gly Glu Val His Cys Gly Thr Asn Val Arg Arg Lys Pro Phe Ser Phe
                645                 650                 655

Lys Trp Trp Asn Met Val Pro
            660

## Description

### Field of the Invention

The present invention relates to a peptidylarginine deiminase 4 inhibitor.

### Background Art

Peptidylarginine deiminase (PAD), a protein modification enzyme widely distributed throughout animal tissues, catalyzes the deimination of a peptidylarginine (protein arginine residue) to convert it into a citrulline residue in a calcium ion-dependent manner (i.e., in the presence of a calcium ion). The deimination of peptidylarginines causes a change in the distribution of positive charges in protein and, as a result, a conformational change occurs in the protein. Therefore, the deimination of a protein exerts a large influence upon the physiological functions of the protein.

PAD was originally found in rodents, and it was demonstrated that three types of PAD were present in the tissues (non-patent documents 1, 2, 3 and 4). Afterward, Nakajima et al. detected the activity of PAD in granulocytes which had been prepared by treating human myelocytic leukemia HL-60 cells with retinoic acid, DMSO or 1,25-dihydroxyvitamin D₃ to induce the differentiation of the cells into granulocyte, and cloned the cDNA of the PAD for analysis (non-patent document 5). As a result, it was generally revealed that the cDNA of the PAD consisted of 2238 bp and encoded 663 amino acid residues, that the amino acid sequence of the PDA was identical by about 50 to 55% to those of known types of human PAD. The PAD identified in human HL-60 cells was named "PAD4" (it was originally named "PAD V", but later renamed "PAD4"). Thereafter, PAD4 was also found to be expressed in human peripheral blood granulocytes (non-patent document 6).

To date, five types of PAD isoforms 1, 2, 3, 4 and 6 have been identified in human (non-patent documents 7, 8, 9, 10, 11, 12, 13, 14, 25 and 26). PAD1 is involved in the differentiation of the skin (non-patent documents 15, 16 and 17), PAD2 is involved in the deimination of myelin basic protein (non-patent documents 18 and 19), and PAD3 is involved in the keratinization of hair follicles (non-patent documents 14, 20 and 21). PAD4, which is found in human HL-60 cells or human peripheral blood (the former name: peptidylarginine deiminase V, PAD V), causes the deimination of nucleophosmin B/23 and histones H2A, H3 and H4 in cells when the calcium level in the cells is increased by treating the cells with a calcium ionophore (non-patent documents 22 and 23). PAD4 has a nuclear localization signal ⁵⁶PPAKKKST⁶³, and therefore is the only PAD isoform among the four types mentioned just above that localizes in the cell nuclei. Based on these findings, PAD4 has been recognized to be a novel histone-modifying enzyme which can act on a chromatin in a calcium ion-dependent manner to regulate the nuclear functions (non-patent document 23). An amino acid sequence comparison that is made among the human PAD isoforms reveals that the isomers share high sequence homology in the C-terminal two-third region. This suggests that the PAD isoforms share the structure of the C-terminal two-third region, in which the active site of PADs is located. Recently, it has been reported that the presence of a single nucleotide polymorphism (SNP) in the PAD4 gene suppresses the mRNA decay to produce excess citrullinated proteins and thereby autoantibodies against the citrullinated proteins are formed in the blood of rheumatoid arthritis patients. This suggests that PAD4 is strongly involved in the development of rheumatoid arthritis (non-patent document 24).

Non-patent document 1: Lamensa, J. W. and Moscarello, M. A. (1993) J. Neurochem., 61, 987-996.
Non-patent document 2: Kubilus, J. and Baden, H. P. (1983) Purification and properties of a brain enzyme which deiminates proteins. Biochim. Biophys. Acta, 745, 285-291.
Non-patent document 3: Kubilus, J. and Baden, H. P. (1983) Purification and properties of a brain enzyme which deiminates proteins. Biochim. Biophys. Acta, 745, 285-291.
Non-patent document 4: Terakawa, H., Takahara, H. and Sugawara, K. (1991) Three types of mouse peptidylarginine deiminase: characterization and tissue distribution. J. Biochem. (Tokyo) 110, 661-666.
Non-patent document 5: Nakashima, K., Hagiwara, T., Ishigami, A., Nagata, S., Asaga, H., Kuramoto, M., Senshu, T. and Yamada, M. (1999) Molecular characterization of peptidylarginine deiminase in HL-60 cells induced by retinoic acid and 1α, 25-dihydroxyvitamin D3. J. Biol. Chem., 274, 27786-27792.
Non-patent document 6: Asaga, H., Nakashima, K. Senshu, T., Ishigami, A. and Yamada, M. (2001) Immunocytochemical localization of peptidylarginine deiminase in human eosinophils and neutrophils. J. Leukocyte Biol., 70, 46-51.
Non-patent document 7: Watanabe, K. and Senshu, T. (1989) J. Biol. Chem., 264, 15255-15260.
Non-patent document 8: Tsuchida, M., Takahara, H., Minami, N., Arai, T., Kobayashi, Y., Tsujimoto, H., Fukazawa, C. and Sugawara, K. (1993) Eur. J. Biochem., 215, 677-685.
Non-patent document 9: Nishij yo, T., Kawada, A., Kanno, T., Shiraiwa, M. and Takahara, H. (1997) J. Biochem. (Tokyo) 121, 868-875.
Non-patent document 10: Yamakoshi, A., Ono, H., Nishijyo, T., Shiraiwa, M. and Takahara, H. (1998) Biochim. Biophys. Acta, 1386, 227-232.
Non-patent document 11: Ishigami, A., Kuramoto, M., Yamada, M., Watanabe, K. and Senshu, T. (1998) FEBS Lett., 433, 113-118.
Non-patent document 12: Rus'd, A. A., Ikejiri, Y., Ono, H., Yonekawa, T., Shiraiwa, M., Kawada, A. and Takahara, H. (1999) Eur. J. Biochem., 259, 660-669.
Non-patent document 13: Nakashima, K., Hagiwara, T., Ishigami, A., Nagata, S., Asaga, H., Kuramoto, M., Senshu, T. and Yamada, M. (1999) Molecular characterization of peptidylarginine deiminase in HL-60 cells induced by retinoic acid and 1α, 25-dihydroxyvitamin D3. J. Biol. Chem., 274, 27786-27792.
Non-patent document 14: Kanno, T., Kawada, A., Yamanouchi, J., Yosida-Noro, C., Yoshiki, A., Siraiwa, M., Kusakabe, M., Manabe, M., Tezuka, T. and Takahara, H. (2000) J. Invest. Dermatol., 115, 813-823.
Non-patent document 15: Senshu, T., Akiyama, K., Kan, S., Asaga, H., Ishigami, A. and Manabe, M . (1995) J. Invest. Dermatol., 105, 163-169.
Non-patent document 16: Senshu, T., Akiyama, K., Ishigami, A. and Nomura, K. (1999) J. Dermatol. Sci., 21, 113-126.
Non-patent document 17: Ishida-Yamamoto, A., Senshu, T., Eady, R. A., Takahashi, H., Shimizu, H., Akiyama, M. and Iizuka, H. (2002) J. Invest. Dermatol., 118, 282-287.
Non-patent document 18: Pritzker LB, Nguyen TA, Moscarello MA. (1997) The developmental expression and activity of peptidylarginine deiminase in the mouse. Neurosci Lett.266, 161-164.
Non-patent document 19: Moscarello MA, Pritzker L, Mastronardi FG, Wood DD. Peptidylarginine deiminase: a candidate factor in demyelinating disease. J Neurochem. 81, 335-43.
Non-patent document 20: Rogers, G., Winter, B., McLaughlan, C., Powell, B. and Nesci, T. (1997) J. Invest. Dermatol., 108, 700-707.
Non-patent document 21: Ohsawa, T., Ishigami, A., Akiyama, K. and Asaga, H. (2001) Biomed. Res., 22, 91-97, Pritzker, L. B., Nguyen, T. A. and Moscarello, M. A. (1999) Neurosci. Lett., 266, 161-164.
Non-patent document 22: Hagiwara, T., Nakashima, K., Hirano, H., Senshu, T. and Yamada, M. (2002) Biochem. Biophys. Res. Commun. 290, 979-983.
Non-patent document 23: Nakashima K, Hagiwara T, Yamada M. (2002) Nuclear localization of peptidylarginine deiminase V and histone deimination in granulocytes. J. Biol. Chem., 277, 49562-49568.
Non-patent document 24: Suzuki, A., Yamada, R., Chang, X., Tokuhiro, S., Sawada, T., Suzuki, M., Nagasaki, M., Nakayama-Hamada, M., Kawaida, R., Ono, M., Ohtsuki, M., Furukawa, H., Yoshino, S., Yukioka, M., Tohma, S., Matsubara, T., Wakitani, S., Teshima, R., Nishioka, Y., Sekine, A., Iida, A., Takahashi, A., Tsunoda, T., Nakamura, Y. and Yamamoto, K. (2003) Functional haplotypes of PADI4, encoding citrullinating enzyme peptidylarginine deiminase 4, are associated with rheumatoid arthritis. Nature Genetics, 34, 395-402.
Non-patent document 25: Wright, P. W. et al. (2003) ePAD, an oocyte and early embryo-abundant peptidylarginine deiminase-like protein that localizes to egg cytoplasmic sheets. Dev Biol. 256, 74-89.
Non-patent document 26: Chavanas, S. et al. (2004) Comparative analysis of the mouse and human peptidylarginine deiminase gene clusters reveals highly conserved non-coding segments and a new human gene, PADI6. Gene 330, 19-27.

### Disclosure of the Invention

### Problems to be Solved by the Invention

The object of the present invention is to design a novel substance capable of inhibiting the enzymatic activity of PAD4 and to develop a new drug against rheumatoid arthritis.

### Means for Solving the Problems

The present inventors determined the three-dimensional structures of the following substances by X-ray diffraction at resolutions of 2.80, 2.60, 2.30, 2.20, 2.25, 2.10, 2.10 and 2.25 angstroms, respectively: PAD4 in the absence of calcium ions (hereinafter sometimes referred to as "Ca²⁺-free PAD4"); mutant PAD4 (C645A) which was inactivated by substitution of Ala for Cys645 (one of the active residues) and had calcium ions bound thereto (hereinafter sometimes referred to as "Ca²⁺-bound PAD4 (C645A)"); and mutant PAD4 (C645A) which was inactivated by the substitution of Ala for Cys645 (one of the active residues) and had calcium ions and the following substrates bound thereto; benzoyl-L-arginine amide (BA); benzoyl-L-arginine ethylester (BAEE); benzoyl-glycyl-L-arginine (BGA); KQTARKSTGG (H3 peptide 1) ; KAPRKQLATK (H3 peptide 2) ; and SGRGKGGKGL (H4 peptide) to form complexes (hereinafter sometimes referred to as "Ca²⁺-bound PAD4 (C645A)-BA complex, Ca²⁺-bound PAD4 (C645A)-BAEE complex, Ca²⁺-bound PAD4 (C645A)-BGA complex, Ca²⁺-bound PAD4 (C645A)-H3 peptide 1 complex, Ca²⁺-bound PAD4 (C645A) -H3 peptide 2 complex, and Ca²⁺-bound PAD4 (C645A)-H4 peptide complex, respectively) (Japanese Patent Application Nos. 2003-358459 and 2004-259125). The conformations of the eight substances were almost the same except for the region surrounding the active site including the calcium-bound sites. A PAD4 molecule had an elongated boot-like shape, and was related with the most proximal molecule in the crystal lattice by a crystallographic two-fold axis to form a functional dimer. The PAD4 molecule was dividable into two domains, the N-terminal domain and the C-terminal domain. The N-terminal domain was further divided into two sub-domains which, when combined; resembled in structure the T-cell surface glycoprotein CD4 that had an immunoglobulin-like structure, with one sub-domain also resembling in structure the DNA-binding domain of p53. The C-terminal domain, on the other hand, was composed of five ββαα-propeller structures and had a negatively charged large groove at the center. The groove included four active residues Asp350, His471, Asp473 and Cys645, and two calcium ions, with the structure around the active residues being similar to those of amidinotransferase (AT) and N(G),N(G)-dimethyl-L-arginine aminidinohydrorase. The structure around the active residues was compared with that of Ca²⁺-free PAD4, revealing that binding of the two calcium ions to the negatively charged large groove caused a significant change in the structure around C645 (A645) and Asp350 and induced a cleft to which a substrate could bind. It was also found that the manner of binding of each calcium ion was distinctly different from that of a well-known EF-hand motif. From these findings, it was demonstrated that PAD4, although being a protein in a superfamily of arginine-modifying enzymes, had an entirely new calcium ion-dependent enzyme-activating mechanism which had not ever been known.

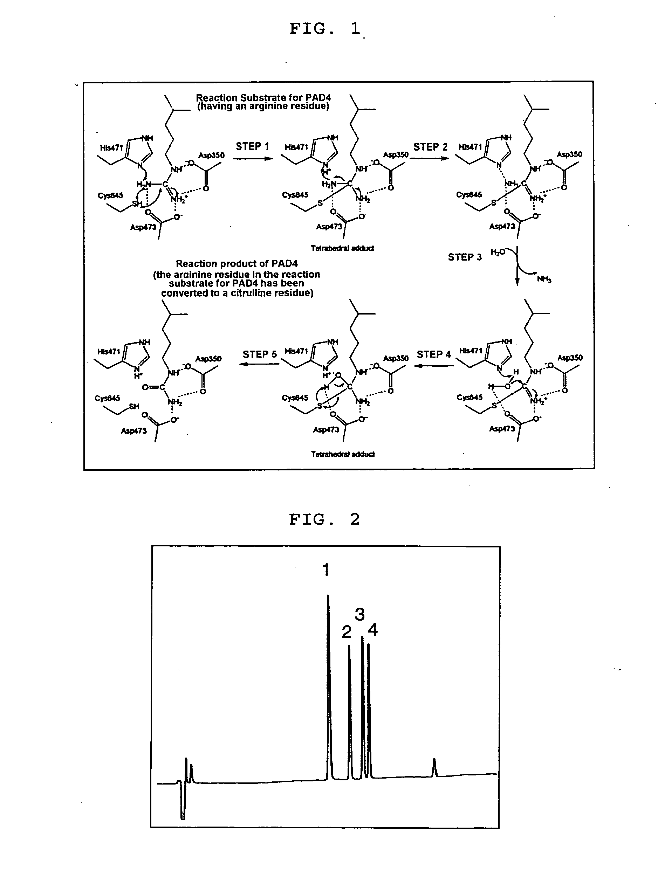

Using programs PSI-BLAST and FUGUE, Shirai et al. speculated that arginine modifying enzymes would share a common fold and proposed a reaction mechanism for deimination of arginine (Shirai, H., Blundell, T. L. and Mizuguchi, K. (2001) A novel superfamily of enzymes that catalyze the modification of guanidino groups. TIBS, 26, 465-468). Following this, Das et al. performed an X-ray crystal structure analysis of arginine deiminase derived from Mycoplasma arginini in complex with a reaction intermediate, and proposed a reaction mechanism for deimination of free L-arginine (Das et al. (2004) Crystal structures of arginine deiminase with covalent reaction intermediates: Implication for catalytic mechanism.) The present inventors made a structural analysis of BA-Ca²⁺ PAD4 (C645A), demonstrating that the deimination reaction mechanism of peptidylarginine (a reaction substrate for PAD4) was consistent with that proposed by Das et al. Therefore, it is assumed that the deimination of peptidylarginine by PAD4 occurs through the two-stage reaction mechanism proposed by Das et al. That is, in the first stage, a thiol group of Cys645 nucleophilically attacks the carbon Cζ in the guanidino group of arginine side-chain to form a tetrahedral adduct. Next, Asp350 and Asp473 form hydrogen bonds and a salt bridge with the substrate, whereby the nucleophilicity of the carbon Cζ in the guanidino group is increased and the binding between the Cζ and NH₂ in the guanidino group are cleaved to produce ammonia. In the second stage, the water molecule activated with His471 nucleophilically attacks the Cζ to form a tetrahedral adduct again. Thereafter, the binding between the Cζ and the sulfur atom Sy in Cys645 is cleaved to produce a peptidylcitrulline residue (the reaction product of PAD4). The PAD4 deimination mechanism proposed by the present inventors is shown in Fig. 1.

Based on the findings mentioned above, the present inventors designed and synthesized novel compounds capable of inhibiting the enzymatic activity of PAD4 and measured the PAD4-inhibition activities of the compounds. As a result, it was found that the compounds possessed a PAD4-inhibition activity, which has led to the accomplishment of the present invention.

The aspects of the present invention are as follows.

(1) A compound represented by the general formula (I) or a salt thereof:

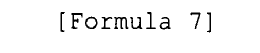

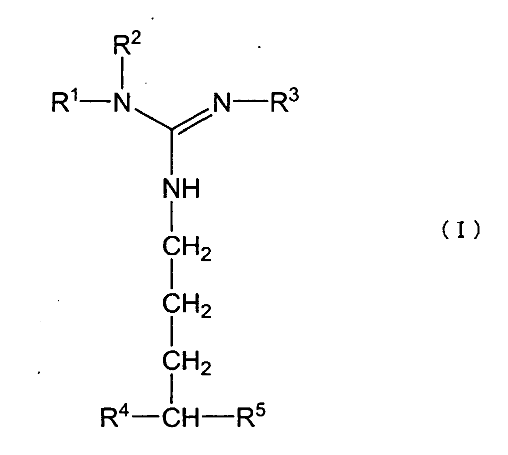

wherein R¹, R² and R³ each independently represent a hydrogen atom or an alkyl group having 1 to 3 carbon atoms, provided that at least one of R¹, R² and R³ does not represent a hydrogen atom; R⁴ represents an amino group which has a substituent; and R⁵ represents a carboxyl group which may have a substituent.
(2) The compound or salt thereof according to item (1), wherein R⁴ represents the following formula:

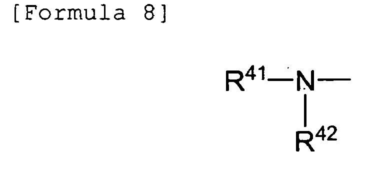

wherein R⁴¹ represents a group represented by R⁴⁰¹CO- where R⁴⁰¹ represents a hydrogen atom, a hydrocarbon group which may have a substituent or a heterocyclic group which may have a substituent, a group represented by R⁴⁰²S (O)ₘ- where R⁴⁰² represents a hydrogen atom, a hydrocarbon group which may have a substituent or a heterocyclic group which may have a substituent, and m is an integer of 1 or 2, a group represented by R⁴⁰⁵N(R⁴⁰⁶)-CHR⁴⁰⁴-CO-[NH-CHR⁴⁰³-CO]ₙ- where R⁴⁰³, R⁴⁰⁴, R⁴⁰⁵ and R⁴⁰⁶ each independently represent a hydrogen atom, a hydrocarbon group which may have a substituent or a heterocyclic group which may have a substituent, and n is an integer of 1 to 50, or a peptidyl group which may have a substituent; and R⁴² represents a hydrogen atom or an alkyl group having 1 to 3 carbon atoms.

(3) The compound or salt thereof according to item (2), wherein R⁴¹ represents a benzoyl group which may have a substituent, a benzoylpeptidyl group which may have a substituent, a dansyl group which may have a substituent or a dansylpeptidyl group which may have a substituent; and R⁴² represents a hydrogen atom.

(4) The compound or salt thereof according to any one of items (1) to (3), wherein R¹, R² and R³ each independently represent a hydrogen atom or a methyl group, provided that at least one of R¹, R² and R³ represents a methyl group.

(5) The compound or salt thereof according to Claim 4, which is a compound represented by the formula (Ia), (Ib) or (Ic) or a salt thereof.

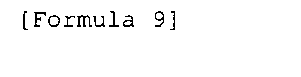

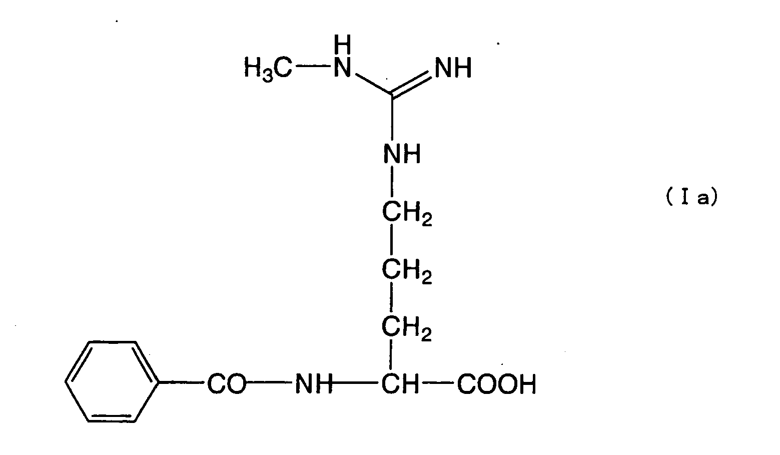

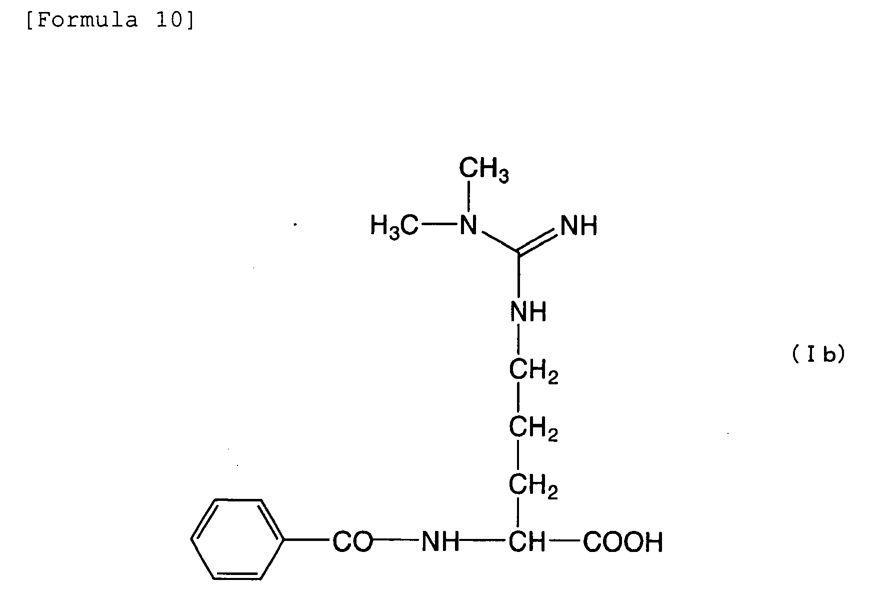

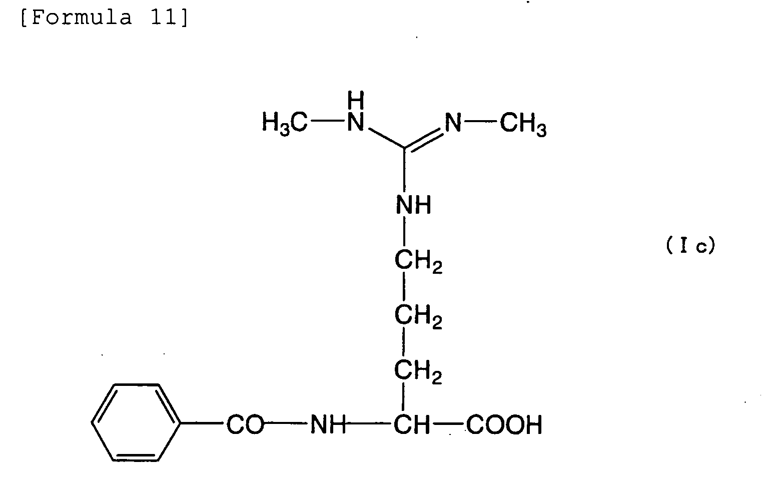

(6) A peptidylarginine deiminase 4 inhibitor comprising as the active ingredient a substance capable of inhibiting any one of the steps 1 to 5 in the reaction mechanism as shown in the following scheme between peptidylarginine deiminase 4 having the amino acid sequence depicted in SEQ ID NO:1 and its reaction substrate.

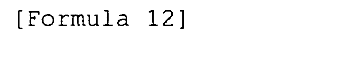

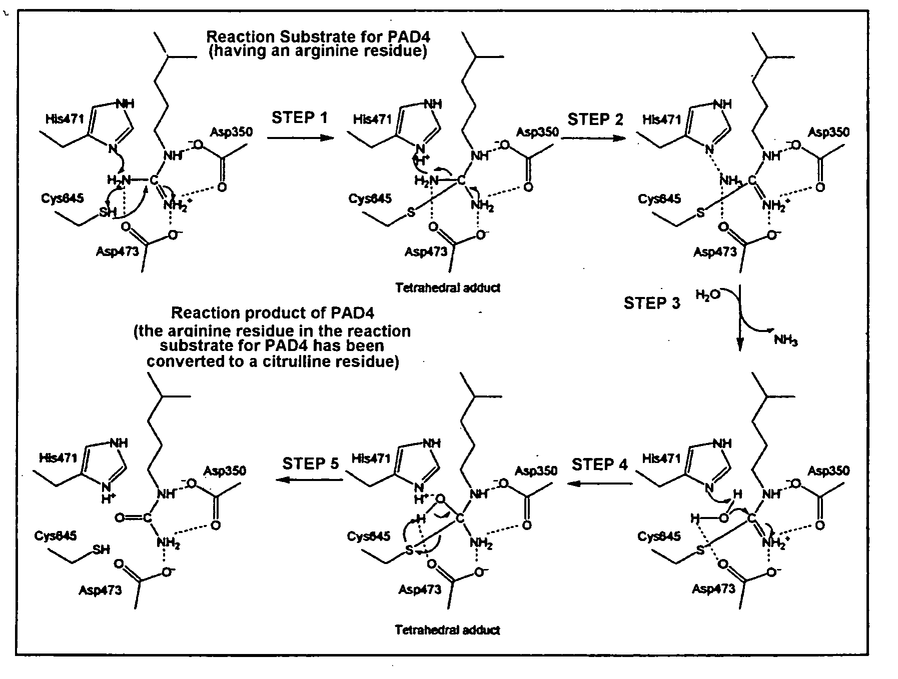

In the scheme, Asp350, His471, Asp473 and Cys645 represent an aspartic acid residue at position 350, a histidine residue at position 471, an aspartic acid residue at position 473 and a cysteine residue at position 645, respectively, in the amino acid sequence depicted in SEQ ID NO:1.
(7) The peptidylarginine deiminase 4 inhibitor according to item (6), wherein the substance capable of inhibiting any one of the steps 1 to 5 in the reaction mechanism between peptidylarginine deiminase 4 having the amino acid sequence depicted in SEQ ID NO:1 and its reaction substrate is an arginine derivative.

(8) A peptidylarginine deiminase 4 inhibitor comprising as the active ingredient an arginine derivative having a substituent on each of the amino and guanidino groups in arginine and optionally having a substituent on the carboxyl group in arginine.

(9) The peptidylarginine deiminase 4 inhibitor according to item (7) or (8), wherein the arginine derivative is a compound or a salt thereof as recited in any one of items (1) to (5).

(10) The peptidylarginine deiminase 4 inhibitor according to any one of items (6) to (9), which is used for the prevention and/or treatment of diseases associated with peptidylarginine deiminases.

(11) The peptidylarginine deiminase 4 inhibitor according to item (10), wherein the diseases associated with peptidylarginine deiminases are selected from the group consisting of rheumatoid arthritis, psoriasis and multiple sclerosis.

As used herein, the term "peptidylarginine deiminase 4" refers to wild type peptidylarginine deiminase 4 having the amino acid sequence depicted in SEQ ID NO:1, and includes analogous substances having a similar biological activity (i.e., the enzymatic activity of catalyzing the reaction for deiminating an arginine residue in a protein into a citrulline residue in the presence of a calcium ion) and which also have amino acid sequences homologous to the amino acid sequence depicted in SEQ ID NO:1.

As used herein, "Boc" represents a t-butoxy group, "Arg" represents arginine, "Tos" represents p-toluenesulfonyl, "Me" represents a methyl group, "ADMA" represents N^{G},N^{G}-dimethyl-L-arginine, "SDMA" represents N^{G}, N^{G'}-dimethyl-L-arginine", and "Bz" represents a benzoyl group.

As used herein, the symbol "-" means a specified range including the numerical values both before and after the symbol as the minimal and maximum values, respectively.

Hereinbelow, the present invention will be described in detail.

### 1. Compounds represented by the general formula (I) or salt thereof

The present invention provides a compound represented by the general formula (I) or a salt thereof.

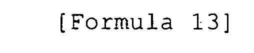

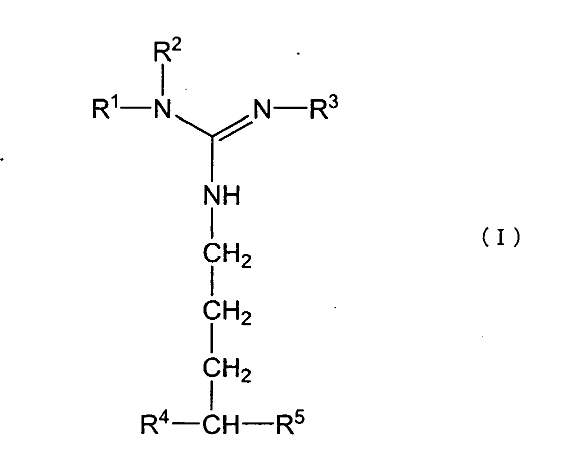

The compound of the general formula (I) or the salt thereof may be of L-, D- or DL-form, but an L-form is effective.

In the general formula (I), R¹, R² and R³ each independently represent a hydrogen atom or an alkyl group having 1 to 3 carbon atoms, provided that at least one of R¹, R² and R³ is not a hydrogen atom. Examples of the alkyl group having 1 to 3 carbon atoms include methyl, ethyl, n-propyl and i-propyl groups.

Preferably, R¹, R² and R³ each independently represent a hydrogen atom or a methyl group, provided that at least one of R¹, R² and R³ is a methyl group.

In the general formula (I), R⁴ represents an amino group which has a substituent. The substituent to be added to the amino group for R⁴ may be of any type, as long as a compound having the substituent can be recognized by PAD4 (i.e., the compound can interact with PAD4). Preferably, the substituent is one having an oxo group (=O) attached to the atom which is directly bound to the nitrogen in the amino group for R⁴. One example of R⁴ is a group represented by the following formula.

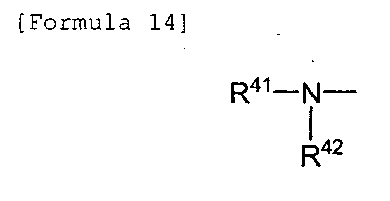

In the formula above, R⁴¹ represents a group represented by R⁴⁰¹CO- where R⁴⁰¹ represents a hydrogen atom, a hydrocarbon group which may have a substituent or a heterocyclic group which may have a substituent, a group represented by R⁴⁰²S (O)ₘ- where R⁴⁰² represents a hydrogen atom, a hydrocarbon group which may have a substituent or a heterocyclic group which may have a substituent, and m is an integer of 1 or 2, a group represented by R⁴⁰⁵N(R⁴⁰⁶)-CHR⁴⁰⁴-CO-[NH-CHR⁴⁰³-CO]ₙ- where R⁴⁰³, R⁴⁰⁴, R⁴⁰⁵ and R⁴⁰⁶ each independently represent a hydrogen atom, a hydrocarbon group which may have a substituent or a heterocyclic group which may have a substituent, and n is an integer of 1 to 50, or a peptidyl group which may have a substituent; and R⁴² represents a hydrogen atom or an alkyl group having 1 to 3 carbon atoms. Examples of the group represented by R⁴⁰⁵N (R⁴⁰⁶) -CHR⁴⁰⁴-CO- and the group represented by -NH-CHR⁴⁰³-CO- include amino acid residues occurring in natural proteins and peptides. Examples of the substituent to be added to the peptidyl group for R⁴¹ include benzoyl and dansyl groups and the like. The benzoyl and dansyl groups and the like may further have a substituent therein. Examples of the substituent for the benzoyl and dansyl groups and the like include a halogen atom (e.g., fluorine, chlorine, bromine, iodine), a hydroxyl group, an alkoxy group having 1 to 6 carbon atoms (e.g., methoxy, ethoxy, propoxy, butoxy, pentoxy), an amino group, a carbamoyl group, an alkoxycarbonyl group having 1 to 6 carbon atoms (e.g., methoxycarbonyl, ethoxycarbonyl, propoxycarbonyl), and a heterocyclic group (examples of the heterocyclic ring in the heterocyclic group include a 5- to 7-membered ring having one sulfur, nitrogen or oxygen atom, a 5-to 6-membered ring having 2 to 4 nitrogen atoms, and a 5- to 6-membered ring having one or two nitrogen atoms and one sulfur or oxygen atom, these heterocyclic rings being optionally fused to a 6-membered ring having one or two nitrogen atoms, a benzene ring or a 5-membered ring having one sulfur atom; specific examples of the heterocyclic group include 2-pyridyl, 3-pyridyl, 4-pyridyl, pyrimidyl, pyrazinyl, pyridazinyl, pyrazolyl, imidazolyl, thiazolyl, isothiazolyl, oxazolyl, isoxazolyl, perido[2,3-d]pyrimidyl, benzopyranyl, 1,8-naphthyridyl, 1,5-naphthyridyl, 1,6-naphthyridyl, 1,7-naphthyridyl, quinolyl, thieno[2,3-b]pyridyl, tetrazolyl, thiadiazolyl, oxadiazolyl, triazinyl, triazolyl, thienyl, pyrrolyl, pyrrolinyl, furyl, pyrrolidinyl, benzothienyl, indolyl, imidazolidinyl, piperidyl, piperidino, piperazinyl, morpholinyl and morpholino). The amino group may be substituted by an alkyl group having 1 to 6 carbon atoms or an acyl group having 1 to 10 carbon atoms. The carbamoyl group may be substituted by an alkyl group having 1 to 6 carbon atoms.

Examples of the hydrocarbon group for R⁴⁰¹, R⁴⁰², R⁴⁰³, R⁴⁰⁴, R⁴⁰⁵ and R⁴⁰⁶ include a saturated chain hydrocarbon group (e.g., a straight-chain or branched alkyl group having 1 to 6 carbon atoms), an unsaturated chain hydrocarbon group (e.g., a straight-chain or branched alkenyl group having 1 to 6 carbon atoms, a straight-chain or branched alkynyl group having 1 to 6 carbon atoms), an alicyclic hydrocarbon group (e.g., a cycloalkyl group having 1 to 6 carbon atoms, a cycloalkenyl group having 1 to 6 carbon atoms, a cycloalkynyl group having 1 to 6 carbon atoms) and an aromatic hydrocarbon group (e.g., phenyl, naphthyl, anthryl and phenanthryl groups).

When R⁴⁰¹, R⁴⁰², R⁴⁰³, R⁴⁰⁴, R⁴⁰⁵ or R⁴⁰⁶ is a hydrocarbon group which may have a substituent, examples of the substituent include a halogen atom (e.g., fluorine, chlorine, bromine, iodine), a hydroxyl group, an alkoxy group having 1 to 6 carbon atoms (e.g., methoxy, ethoxy, propoxy, butoxy, pentoxy), an amino group, a carbamoyl group, an alkoxycarbonyl group having 1 to 6 carbon atoms (e.g., methoxycarbonyl, ethoxycarbonyl, propoxycarbonyl), and a heterocyclic group (examples of the heterocyclic ring in the heterocyclic group include a 5- to 7-membered ring having one sulfur, nitrogen or oxygen atom, a 5- to 6-membered ring having 2 to 4 nitrogen atoms, and a 5- to 6-membered ring having one or two nitrogen atoms and one sulfur or oxygen atom, these heterocyclic rings being optionally fused to a 6-membered ring having one or two nitrogen atoms, a benzene ring or a 5-membered ring having one sulfur atom; specific examples of the heterocyclic group include 2-pyridyl, 3-pyridyl, 4-pyridyl, pyrimidyl, pyrazinyl, pyridazinyl, pyrazolyl, imidazolyl, thiazolyl, isothiazolyl, oxazolyl, isoxazolyl, pyrido[2,3-d]pyrimidyl, benzopyranyl, 1,8-naphthyridyl, 1,5-naphthyridyl, 1,6-naphthyridyl, 1,7-naphthyridyl, quinolyl, thieno[2,3-b]pyridyl, tetrazolyl, thiadiazolyl, oxadiazolyl, triazinyl, triazolyl, thienyl, pyrrolyl, pyrrolinyl, furyl, pyrrolidinyl, benzothienyl, indolyl, imidazolidinyl, piperidyl, piperidino, piperazinyl, morpholinyl and morpholino). The amino group may be substituted by an alkyl group having 1 to 6 carbon atoms or an acyl group having 1 to 10 carbon atoms. The carbamoyl group may be substituted by an alkyl group having 1 to 6 carbon atoms.

Examples of the heterocyclic ring in the heterocyclic group for R⁴⁰¹, R⁴⁰², R⁴⁰³, R⁴⁰⁴, R⁴⁰⁵ or R⁴⁰⁶ include a 5- to 7-membered ring having one sulfur, nitrogen or oxygen atom, a 5- to 6-membered ring having 2 to 4 nitrogen atoms, and a 5- to 6-membered ring having one or two nitrogen atoms and one sulfur or oxygen atom, these heterocyclic rings being optionally fused to a 6-membered ring having one or two nitrogen atoms, a benzene ring or a 5-membered ring having one sulfur atom. Specific examples of the heterocyclic group include 2-pyridyl, 3-pyridyl, 4-pyridyl, pyrimidyl, pyrazinyl, pyridazinyl, pyrazolyl, imidazolyl, thiazolyl, isothiazolyl, oxazolyl, isoxazolyl, pyrido[2,3-d]pyrimidyl, benzopyranyl, 1,8-naphthyridyl, 1,5-naphthyridyl, 1,6-naphthyridyl, 1,7-naphthyridyl, quinolyl, thieno[2,3-b]pyridyl, tetrazolyl, thiadiazolyl, oxadiazolyl, triazinyl, triazolyl, thienyl, pyrrolyl, pyrrolinyl, furyl, pyrrolidinyl, benzothienyl, indolyl, imidazolidinyl, piperidyl, piperidino, piperazinyl, morpholinyl and morpholino.

When R⁴⁰¹, R⁴⁰², R⁴⁰³, R⁴⁰⁴, R⁴⁰⁵ or R⁴⁰⁶ is a heterocyclic group which may have a substituent, examples of the substituent include a halogen atom (e.g., fluorine, chlorine, bromine, iodine), a hydroxyl group, an alkyl group having 1 to 6 carbon atoms (e.g., methyl, ethyl, n-propyl, i-propyl), an alkoxy group having 1 to 6 carbon atoms (e.g., methoxy, ethoxy, propoxy, butoxy, pentoxy), an amino group, a carbamoyl group, an alkoxycarbonyl group having 1 to 6 carbon atoms (e.g., methoxycarbonyl, ethoxycarbonyl, propoxycarbonyl), and a heterocyclic ring as mentioned above. The amino group may be substituted by an alkyl group having 1 to 6 carbon atoms or an acyl group having 1 to 10 carbon atoms. The carbamoyl group may be substituted by an alkyl group having 1 to 6 carbon atoms.

Examples of the alkyl group having 1 to 3 carbon atoms for R⁴² include methyl, ethyl, n-propyl and i-propyl groups.

Preferably, R⁴¹ is a benzoyl group which may have a substituent, a benzoylpeptidyl group which may have a substituent, a dansyl group which may have a substituent or a dansylpeptidyl group which may have a substituent, and R⁴² is a hydrogen atom.
In the general formula (I), R⁵ is a carboxyl group which may have a substituent. When R⁵ is a carboxyl group which has a substituent, the substituent may be of any type. For example, in order to increase the inhibitory activity against PAD4, R⁵ is preferably a group represented by -COOR⁵¹ wherein R⁵¹ represents an alkyl group having 1 to 20 carbon atoms, a group represented by -COO-{R⁵⁴N(R⁵⁵)-CHR⁵³-CO-[NH-CHR⁵²-CO]ₚ-} wherein R⁵², R⁵³, R⁵⁴ and R⁵⁵ each independently represent a hydrogen atom, a hydrocarbon group which may have a substituent or a heterocyclic group which may have a substituent, and p is an integer of 1 to 50, or like groups. Examples of the group represented by R⁵⁴N(R⁵⁵)-CHR⁵³-CO- and the group represented by -NH-CHR⁵²-CO- include amino acid residues occurring in natural proteins and peptides.
The alkyl group for R⁵¹ may be either straight-chain or branched alkyl group having 1 to 20 carbon atoms, and may specifically be exemplified by methyl, ethyl, n-propyl, i-propyl, n-butyl, i-butyl, sec-butyl, tert-butyl, pentyl, isopentyl, neopentyl, hexyl, heptyl, octyl, nonyl and decyl groups.
Examples of the hydrocarbon group for R⁵², R⁵³, R⁵⁴ and R⁵⁵ include a saturated chain hydrocarbon group (e.g., a straight-chain or branched alkyl group having 1 to 6 carbon atoms), an unsaturated chain hydrocarbon group (e.g., a straight-chain or branched alkenyl group having 1 to 6 carbon atoms, a straight-chain or branched alkynyl group having 1 to 6 carbon atoms), an alicyclic hydrocarbon group (e.g., a cycloalkyl group having 1 to 6 carbon atoms, a cycloalkenyl group having 1 to 6 carbon atoms, a cycloalkynyl group having 1 to 6 carbon atoms) and an aromatic hydrocarbon group (e.g., phenyl, naphthyl, anthryl and phenanthryl groups).
When R⁵², R⁵³, R⁵⁴ or R⁵⁵ is a hydrocarbon group which may have a substituent, examples of the substituent include a halogen atom (e.g., fluorine, chlorine, bromine, iodine), a hydroxyl group, an alkoxy group having 1 to 6 carbon atoms (e.g., methoxy, ethoxy, propoxy, butoxy, pentoxy), an amino group, a carbamoyl group, an alkoxycarbonyl group having 1 to 6 carbon atoms (e.g., methoxycarbonyl, ethoxycarbonyl, propoxycarbonyl), and a heterocyclic group (examples of the heterocyclic ring in the heterocyclic group include a 5- to 7-membered ring having one sulfur, nitrogen or oxygen atom, a 5- to 6-membered ring having 2 to 4 nitrogen atoms, and a 5- to 6-membered ring having one or two nitrogen atoms and one sulfur or oxygen atom, these heterocyclic rings being optionally fused to a 6-membered ring having one or two nitrogen atoms, a benzene ring or a 5-membered ring having one sulfur atom; specific examples of the heterocyclic group include 2-pyridyl, 3-pyridyl, 4-pyridyl, pyrimidyl, pyrazinyl, pyridazinyl, pyrazolyl, imidazolyl, thiazolyl, isothiazolyl, oxazolyl, isoxazolyl, pyrido[2,3-d]pyrimidyl, benzopyranyl, 1,8-naphthyridyl, 1,5-naphthyridyl, 1,6-naphthyridyl, 1,7-naphthyridyl, quinolyl, thieno[2,3-b]pyridyl, tetrazolyl, thiadiazolyl, oxadiazolyl, triazinyl, triazolyl, thienyl, pyrrolyl, pyrrolinyl, furyl, pyrrolidinyl, benzothienyl, indolyl, imidazolidinyl, piperidyl, piperidino, piperazinyl, morpholinyl and morpholino). The amino group may be substituted by an alkyl group having 1 to 6 carbon atoms or an acyl group having 1 to 10 carbon atoms. The carbamoyl group may be substituted by an alkyl group having 1 to 6 carbon atoms.
The heterocyclic ring in the heterocyclic group for R⁵², R⁵³, R⁵⁴ or R⁵⁵ may be exemplified by a 5- to 7-membered ring having one sulfur, nitrogen or oxygen atom, a 5- to 6-membered ring having 2 to 4 nitrogen atoms, and a 5- to 6-membered ring having one or two nitrogen atoms and one sulfur or oxygen atom, these heterocyclic rings being optionally fused to a 6-membered ring having one or two nitrogen atoms, a benzene ring or a 5-membered ring having one sulfur atom. Specific examples of the heterocyclic group include 2-pyridyl, 3-pyridyl, 4-pyridyl, pyrimidyl, pyrazinyl, pyridazinyl, pyrazolyl, imidazolyl, thiazolyl, isothiazolyl, oxazolyl, isoxazolyl, pyrido[2,3-d]pyrimidyl, benzopyranyl, 1,8-naphthyridyl, 1,5-naphthyridyl, 1,6-naphthyridyl, 1,7-naphthyridyl, quinolyl, thieno[2,3-b]pyridyl, tetrazolyl, thiadiazolyl, oxadiazolyl, triazinyl, triazolyl, thienyl, pyrrolyl, pyrrolinyl, furyl, pyrrolidinyl, benzothienyl, indolyl, imidazolidinyl, piperidyl, piperidino, piperazinyl, morpholinyl and morpholino.
When R⁵², R⁵³, R⁵⁴ or R⁵⁵ is a heterocyclic group which may have a substituent, examples of the substituent include a halogen atom (e.g., fluorine, chlorine, bromine, iodine), a hydroxyl group, an alkyl group having 1 to 6 carbon atoms (e.g., methyl, ethyl, n-propyl and i-propyl), an alkoxy group having 1 to 6 carbon atoms (e.g., methoxy, ethoxy, propoxy, butoxy, pentoxy), an amino group, a carbamoyl group, an alkoxycarbonyl group having 1 to 6 carbon atoms (e.g., methoxycarbonyl, ethoxycarbonyl, propoxycarbonyl), and a heterocyclic group as mentioned above. The amino group may be substituted by an alkyl group having 1 to 6 carbon atoms or an acyl group having 1 to 10 carbon atoms. The carbamoyl group may be substituted by an alkyl group having 1 to 6 carbon atoms.
Specific examples of the compound represented by the general formula (I) include compounds represented by the following formulae (Ia), (Ib) and (Ic).

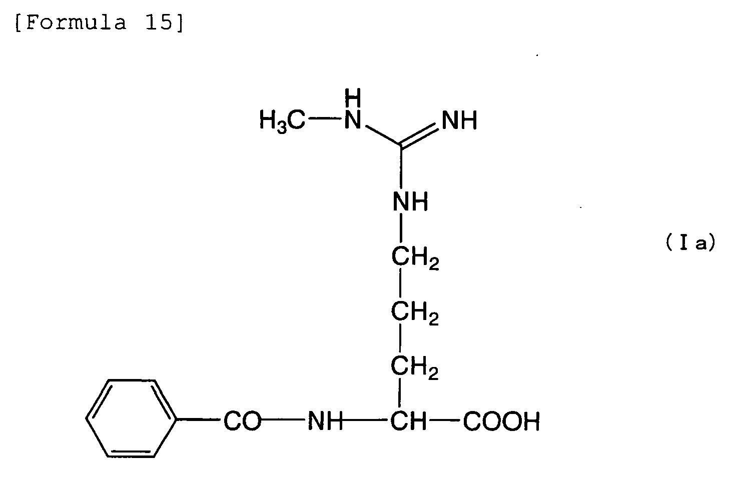

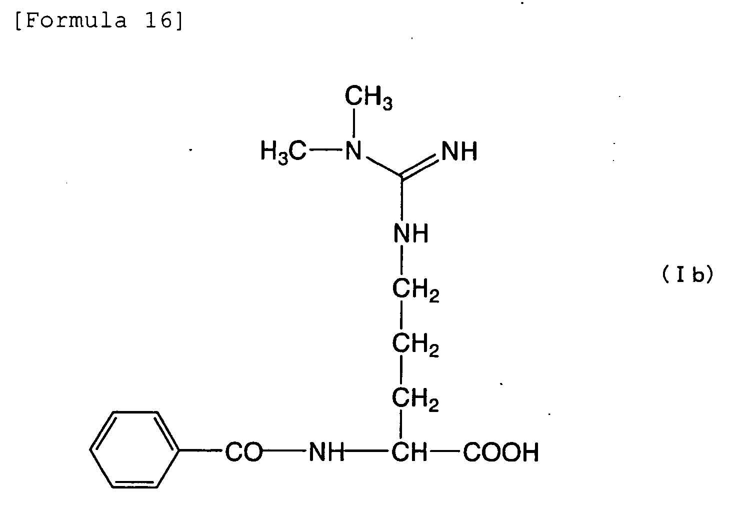

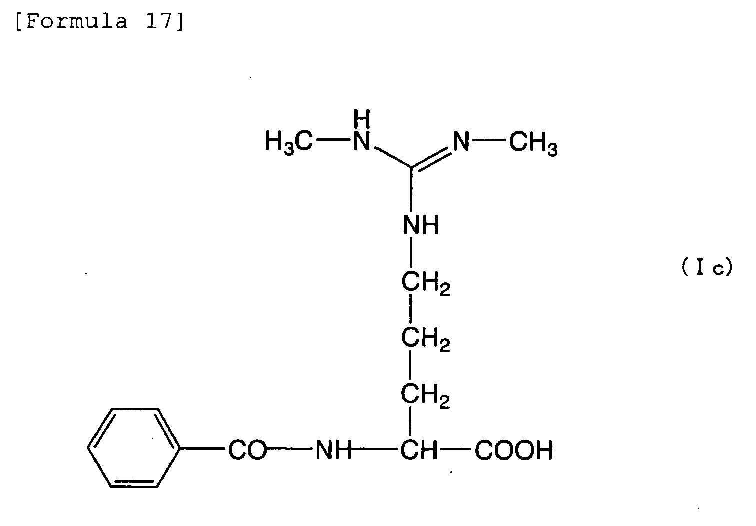

The compound represented by the formula (Ia) is Bz-Arg (mono-methyl). The compound represented by the formula (Ib) is Bz-ADMA. The compound represented by the formula (Ic) is Bz-SDMA.
The compound represented by the general formula (I) can be synthesized starting from commercially available arginine or an arginine derivative represented by the following formula.

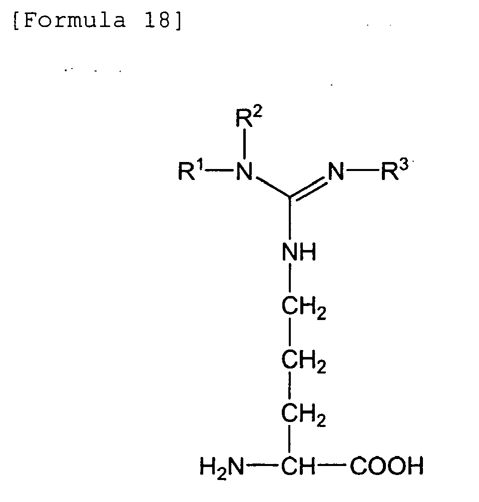

wherein R¹, R² and R³ each independently represent a hydrogen atom or an alkyl group having 1 to 3 carbon atoms, provided that at least one of R¹, R² and R³ is not a hydrogen atom.
A compound of the general formula (I) wherein R⁴ is a group represented by R⁴⁰¹-CO-NH- where R⁴⁰¹ represents a hydrogen atom, a hydrocarbon group which may have a substituent or a heterocyclic group which may have a substituent, and wherein R⁵ represents a carboxyl group, can be produced by acylation of the starting material (i.e., arginine or the arginine derivative mentioned above) with a symmetric acid anhydride represented by R⁴⁰¹CO-O-COR⁴⁰¹ or by benzoylation of the starting material with Bz₂O (benzoic anhydride). The benzoylation reaction can be performed in any known manner. For example, the benzoylation reaction may be performed in an inert solvent in the presence of a base. The inert solvent to be used in this reaction may be exemplified by dimethylformamide (DMF), dimethyl sulfoxide (DMSO) and tetrahydrofuran (THF), which may be mixed with water or with themselves. As for the base, sodium hydrogencarbonate or potassium hydrogencarbonate may be used so that the pH of the reaction solution is adjusted to about 10 or lower in view of the fact that the pKa value of the guanidino skeleton in the arginine side chain is about 12. The reaction temperature is preferably about 0 to 37°C, and the reaction time is preferably about 10 minutes to about 24 hours. The amount of Bz₂O to be used is preferably about 1 to 1.2 moles per mole of arginine or the arginine derivative (starting material) to be used.
Speaking of a compound of the general formula (I) wherein R⁴ is a group represented by R⁴⁰²-S(O)ₘ-NH- where R⁴⁰² represents a hydrogen atom, a hydrocarbon group which may have a substituent or a heterocyclic group which may have a substituent, and m is an integer of 1 or 2, and wherein R⁵ represents a carboxyl group, it can, if m = 2, be produced by dansylation of the starting materinal (i.e., arginine or the arginine derivative mentioned above) with DNS-Cl (dansyl chloride). The dansylation reaction can be performed in any known manner (B.S. Hartley, V. Massey, Biochim. Biophys. Acta, 21, 58 (1956)). For example, the dansylation reaction may be performed in an inert solvent in the presence of a base. The inert solvent to be used in this reaction may be exemplified by acetone, dimethylformamide (DMF), dimethyl sulfoxide (DMSO) and tetrahydrofuran (THF), which may be mixed with water or with themselves. As for the base, sodium hydrogencarbonate or potassium hydrogencarbonate may be used so that the pH of the reaction solution is adjusted to about 10 or lower in view of the fact that the pKa value of the guanidino skeleton in the arginine side chain is about 12. The reaction temperature is preferably about 0 to 37 °C, and the reaction time required is preferably about 10 minutes to about 24 hours. The amount of DNS-C1 to be used is preferably about 1 to 1.2 moles per mole of arginine or the arginine derivative (starting material) and its concentration is desirably about 5 mM.
A compound of the general formula (I) wherein R⁴ is a group represented by R⁴⁰⁵N(R⁴⁰⁶)-CHR⁴⁰⁴-CO-[NH-CHR⁴⁰³-CO]ₙ-NH- where R⁴⁰³, R⁴⁰⁴, R⁴⁰⁵ and R⁴⁰⁶ each independently represent a hydrogen atom, a hydrocarbon group which may have a substituent or a heterocyclic group which may have a substituent and n is an integer of 1 or 50, and wherein R⁵ represents a carboxyl group, can be produced by the following exemplary method. First, arginine or the arginine derivative described above (starting material) is butyloxycarbonylated with Boc₂O (t- butyloxycarbonylated symmetric acid anhydride) in the same manner as in the benzoylation mentioned above. Boc-Arg or a derivative thereof produced by this reaction is then treated with p-toluenesulfonyl chloride to tosylate the guanidino group in the side chain in accordance with a known method (J. Ramachandran, C.H. Li, J. Org. Chem., 27, 4006 (1962)). The peptide can be produced by using this derivative according to a known method, or the so-called solid-phase synthesis method for peptide (awarded the Nobel Prize in Chemistry) (R.B. Merrifield, J. Am. Chem. Soc., 85, 2149 (1963)).
A compound of the general formula (I) wherein R⁴ is a group represented by R⁴¹-NH- where R⁴¹ represents a benzoylpeptidyl group which may have a substituent and wherein R⁵ represents a carboxyl group, can be synthesized by the following exemplary method.
First, a peptide chain is synthesized in accordance with the known Fmoc solid-phase synthesis method (Atherton, E. and Sheppard, R.C., 1989, Solid Phase Synthesis. A Practical Approach., IPF Press, Oxford, UK) . In this case, Asp, Glu, Lys and Arg are used in such forms that the carboxyl group in the side chain can be cleaved with HF rather than TFA and respective examples are Fmoc-Asp(OcHex), Fmoc-Glu(OcHex), Fmoc-Lys(Cl-Z) and Fmoc-Arg(Tos). After removing the Fmoc group (tail end) at the N-terminal amino acid residue, benzoylation is performed with Bz₂O (benzoic anhydride) in any known manner as described above. Next, the resin having the desired peptide attached thereto is treated with TFA in the presence of a scavenger reagent (e.g., ethanedithiol; thioanisole) and the released peptide of interest is purified by HPLC or the like. The peptide in the free form is dissolved in a solvent (e.g., DMF), mixed with one equivalent of 1-ethyl-3-(3-dimethylaminopropyl)carbodiimide under ice-cooling to produce an anhydride of the peptide, to which Arg or an Arg derivative is then added. As for the base to be added, sodium hydrogencarbonate or potassium hydrogencarbonate may be used so that the pH of the reaction solution is adjusted to about 10 or lower in view of the fact that the pKa value of the guanidino skeleton in the arginine side chain is about 12. The reaction temperature is preferably about 0 to 37°C, and the reaction time is preferably about 10 minutes to about 24 hours. Finally, the peptide thus produced is treated with HF (anhydrous hydrogen fluoride) to remove all of the protective groups other than the benzoyl group and the peptide is then purified.
A compound of the general formula (I) wherein R⁴ is a group represented by R⁴¹-NH- where R⁴¹ represents a dansylpeptidyl group which may have a substituent and wherein R⁵ represents a carboxyl group, can be synthesized according to the method described above, except that the dansylation is performed by adding dansyl chloride after the removal of the Fmoc group.
Speaking of a compound of the general formula (I) wherein R⁴ is a group represented by R⁴⁰¹-CO-NR⁴² where R⁴⁰¹ represents a hydrogen atom, a hydrocarbon group which may have a substituent or a heterocyclic group which may have a substituent, and wherein R⁵ represents a carboxyl group, it can, if R⁴² is a methyl group(-CH₃), be synthesized by treating an N^{α}-methyl form of arginine or the arginine derivative (as a starting material) with a symmetric acid anhydride represented by R⁴⁰¹CO-O-COR⁴⁰¹. For example, the reaction may be performed in an inert solvent in the presence of a base. The inert solvent to be used in this reaction may be exemplified by dimethylformamide (DMF), dimethyl sulfoxide (DMSO) and tetrahydrofuran (THF), which may be mixed with water or with themselves. As for the base, sodium hydrogencarbonate or potassium hydrogencarbonate may be used so that the pH of the reaction solution is adjusted to about 10 or lower in view of the fact that the pKa value of the guanidino skeleton in the arginine side chain is about 12. The reaction temperature is preferably about 0 to 37°C, and the reaction time is preferably about 10 minutes to about 24 hours. The amount of the symmetric acid anhydride to be used is preferably about 1 to 1.2 moles per mole of the N^{α}-methyl form of arginine or the arginine derivative (starting material).
As the starting material for the synthesis of a compound of the general formula (I) wherein R⁴² is a methyl group (CH₃-), Boc-N-Me-Arg(Tos)-OH is commercially available from BACHEM AG. This compound is treated with trifluoroacetic acid to remove the Boc group, thereby producing N-Me-Arg(Tos)-OH (Text for Biochemical Experiments Vol. 1, Chemistry of Proteins IV -Chemical Modification and Peptide Synthesis-, p.234, ed. the Society of Biochemistry, Japan, published by Tokyo Kagaku-Dojin, Tokyo, Japan). This product may be treated with a symmetric acid anhydride or Bz₂O to modify the α-amino group in the methyl form in various manners.
A compound of the general formula (I) wherein the guanidino group in the side chain is methylated and the α-amino group is methylated can be synthesized as follows. First, commercially available Arg (mono-methyl), ADMA or SDMA is butyloxycarbonylated (T. Nagasawa, K. Kuroiwa, K. Narita, Y. Isowa, Bull. Chem. Soc. Jpn., 46, 1269 (1973)) to produce Boc-Arg (mono-methyl), Boc-ADMA or Boc-SDMA. Next, the methylated guanidino group in the side chain is further tosylated (J. Ramachandran, C. H. Li, J. Org. Chem. , 27, 4006 (1962)) to prepare the respective tosylated form, Boc-Arg (mono-methyl,Tos), Boc-ADMA(Tos) or Boc-SDMA (Tos). This compound is treated with trifluoroacetic acid to remove the Boc group to produce Arg(mono-methyl,Tos), ADMA(Tos) or SDMA(Tos). The resulting product is used as a starting material and converted into an N-benzylideneamino acid, which is then reduced into an N-benzylated compound. The N-benzylated compound is methylated with formalin and formic acid and then subjected to catalytic reduction to remove the benzyl group, thereby producing N-Me-Arg (mono-methyl,Tos), N-Me-ADMA (Tos) or N-Me-SDMA(Tos) (P. Quitt, J. Hellerbach, K. Volger, Helv. Chim. Acta, 46, 327 (1963)). This product is treated with HF as described above (S. Sakakibara, Y. Shimonishi, Y. Kishida, M. Okada, H. Sugihara, Bull. Chem. Soc. Jpn, 40, 2164 (1967)) to produce N-Me-Arg (mono-methyl), N-Me-ADMA or N-Me-SDMA. The compound may be used as a starting material which is treated with a symmetric acid anhydride or Bz₂O to modify the α-amino group in the methyl form in various manners.
Speaking of a compound of the general formula (I) wherein R⁴ is a group represented by R⁴⁰²-S (0) ₘ-NR⁴²- where R⁴⁰² represents a hydrogen atom, a hydrocarbon group which may have a substituent or a heterocyclic group which may have a substituent, and m is an integer of 1 or 2, and wherein R⁵ represents a carboxyl group, it can, if m = 2 and R⁴² is a methyl group (CH₃-), be produced by dansylation of an N^{α}-methyl form of a starting substance (i.e., arginine or the arginine derivative mentioned above) with DNS-Cl (dansyl chloride). The dansylation reaction can be performed in any known manner (B.S. Hartley, V. Massey, Biochim. Biophys. Acta, 21, 58 (1956)). For example, the dansylation reaction may be performed in an inert solvent in the presence of a base. The inert solvent to be used in this reaction may be exemplified by acetone, dimethylformamide (DMF), dimethyl sulfoxide (DMSO) and tetrahydrofuran (THF), which may be mixed with water or with themselves. As for the base, sodium hydrogencarbonate or potassium hydrogencarbonate may be used so that the pH of the reaction solution is adjusted to about 10 or lower in view of the fact that the pKa value of the guanidino skeleton in the arginine side chain is about 12. The reaction temperature is preferably about 0 to 37°C, and the reaction time is preferably about 10 minutes to about 24 hours. The amount of DNS-Cl to be used is preferably about 1 to 1.2 moles per mole of the N^{α}-methyl form of arginine or the arginine derivative (starting material) and its concentration is desirably about 5 mM.
Speaking of a compound of the general formula (I) wherein R⁴ is a group represented by R⁴⁰⁵N(R⁴⁰⁶)-CHR⁴⁰⁴-CO-[NH-CHR⁴⁰³-CO]ₙ-NR⁴²- where R⁴⁰³, R⁴⁰⁴, R⁴⁰⁵ and R⁴⁰⁶ each independently represent a hydrogen atom, a hydrocarbon group which may have a substituent or a heterocyclic group which may have a substituent and n is an integer of 1 or 50, and wherein R⁵ represents a carboxyl group, it can, if R⁴² is a methyl group (CH₃-), be produced by butyloxylcarbonylation of an N^{α}-methyl form of a starting material (i.e., arginine or the arginine derivative mentioned above) with Boc₂O (a t-butyloxycarbonylated symmetric acid anhydride) in the same manner as in the benzoylation mentioned above. The N^{α}-methyl form of Boc-Arg or the derivative thereof produced by this reaction is then treated with p-toluenesulfonyl chloride to tosylate the guanidino group in the side chain in accordance with a known method (J. Ramachandran, C . H . Li, J. Org. Chem., 27, 4006 (1962)). The peptide can be produced by using this derivative according to the known, so-called solid phase synthesis method for peptide (awarded the Nobel Prize in Chemistry) (R.B. Merrifield, J. Am. Chem. Soc., 85, 2149 (1963)).
Speaking of a compound of the general formula (I) wherein R⁴ is a group represented by R⁴¹-NR⁴²- where R⁴¹ represents a benzoylpeptidyl group which may have a substituent and wherein R⁵ represents a carboxyl group, it can, if R⁴² is a methyl group (CH₃-), be synthesized by the following exemplary method.
First, a peptide chain is synthesized in accordance with the known Fmoc solid-phase synthesis method (Atherton, E. and Sheppard, R.C., 1989, Solid Phase Synthesis. A Practical Approach., IPF Press, Oxford, UK). In this case, Asp, Glu, Lys and Arg are used in such forms that the carboxyl group in the side chain can be cleaved with HF rather than TFA and respective examples are Fmoc-Asp(OcHex), Fmoc-Glu(OcHex), Fmoc-Lys(Cl-Z) and Fmoc-Arg(Tos). After removing the Fmoc group (tail end) at the N-terminal amino acid residue, benzoylation is performed with Bz₂O (benzoic anhydride) in any known manner as described above. Next, the resin having the desired peptide attached thereto is treated with TFA in the presence of a scavenger reagent (e.g., ethanedithiol, thioanisole) and the released peptite of interest is purified by HPLC or the like. The peptide in the free form is dissolved in a solvent (e.g., DMF), mixed with one equivalent of 1-ethyl-3-(3-dimethylaminopropyl)carbodiimide under ice-cooling to produce an anhydride of the peptide, to which N^{α}-methylated Arg or an N^{α}-methylated Arg derivative is then added. As for the base to be added, sodium hydrogencarbonate or potassium hydrogencarbonate may be used so that the pH of the reaction solution is adjusted to about 10 or lower in view of the fact that the pKa value of the guanidino skeleton in the arginine side chain is about 12. The reaction temperature is preferably about 0 to 37°C, and the reaction time is preferably about 10 minutes to about 24 hours. Finally, the peptide thus produced is treated with HF (anhydrous hydrogen fluoride) to remove all of the protective groups other than the benzoyl group and the peptide is then purified.
Speaking of a compound of the general formula (I) wherein R⁴ is a group represented by R⁴¹-NR⁴²- where R⁴¹ represents a dansylpeptidyl group which may have a substituent and wherein R⁵ represents a carboxyl group, it can, if R⁴² is a methyl group (CH₃-), be synthesized according to the method described above, except that the dansylation is performed by adding dansyl chloride after the removal of the Fmoc group.
Hereinafter, the method for introducing a substituent into the carboxyl group for R⁵ will be described briefly. For example, if it is desired to introduce an alkyl group (e.g., methyl group, ethyl group) or a benzyl group into the carboxyl group for R⁵, esterification of Arg or a derivative thereof is performed in accordance with a known method (H. Yajima, Y. Kiso, K. Kitagawa, Chem. Pharm. Bull., 22, 1079 (1974) and M. Brenner, W. Huber, Helv. Chim. Acta, 36, 1109 (1953)). The material thus produced may be used as a starting material and subjected to reaction (e.g., benzoylation) in the same manner as in the benzoylation reaction or the like described above. In this manner, various compounds can be synthesized.
Hereinafter, the method for synthesis of a compound of the general formula (I) wherein R⁵ is -COO-[NR⁵⁴-CHR⁵³-CO- (NH-CHR⁵²CO-)ₚ] will be described briefly. If R⁵⁴ is a hydrogen atom, a protected amino acid resin having a C-terminal amino acid bound to Merrifield resin (polystyrene resin) is prepared according to the Gisin method (B. F. Gisin, Helv. Chem.Acta, 56, 1476 (1973)). Using the protected amino acid resin as a starting material, the peptide solid-phase synthesis (R.B. Merrifield, J. Am. Chem. Soc., 85, 2149 (1963)) is repeated p-1 times and Boc-NR⁵⁴-CHR⁵³-COOH is then condensed. In the next step, Boc-Arg(Tos) (Peptide Institute, Inc., Minoo-shi, Osaka, Japan) or an Arg derivative which has been treated with p-toluenesulfonyl chloride to tosylate the guanidino group in the side chain (J. Ramachandran, C.H. Li, J. Org. Chem., 27, 4006 (1962)) is further bound by the peptide solid phase synthesis method. The resulting product is treated with hydrogen fluoride (HF) (S. Sakakibara, Y. Shimonishi, Y. Kishida, M. Okada, H. Sugihara, Bull. Chem. Soc. Jpn, 40, 2164 (1967)) to produce the desired product.
A compound of the general formula (I) wherein R⁵ is -COO-[NR⁵⁴-CHR⁵³-CO- (NH-CHR⁵²CO-)ₚ] where R⁵⁴ is a methyl group, can be produced as follows: N-Me-Arg(mono-methyl,Tos), N-Me-ADMA(Tos) or N-Me-SDMA(Tos) described above is butyloxycarbonylated to produce Boc-N-Me-Arg(mono-methyl,Tos), Boc-N-Me-ADMA (Tos) orBoc-N-Me-SDMA(Tos), respectively, which is then introduced into a desired site by the peptide solid phase synthesis method described above to prepare the desired product.
If the compound of the general formula (I) has an acidic functional group (e.g., a carboxyl group), it may be provided in the form of a salt with a base (e.g., a pharmaceutically acceptable base) in a conventional manner. Example of such include salts with sodium, potassium, aluminum and calcium. If the compound of the general formula (I) has a basic functional group (e.g., an amino group, a mono-substituted amino group), it may be provided in the form of a salt with an acid (e.g., a pharmaceutically acceptable acid) in a conventional manner. Examples of such salt include a hydrochloride, a sulfate, an acetate and a fumarate.
The compound of the general formula (I) or a salt thereof can be used as a peptidylarginine deiminase 4 inhibitor.
2. Peptidylarginine deiminase 4 (PAD4) inhibitor
The present invention provides a peptidylarginine deiminase 4 inhibitor comprising as the active ingredient a substance capable of inhibiting any one of the steps 1 to 5 in the reaction mechanism as shown in the following scheme between peptidylarginine deiminase 4 having the amino acid sequence depicted in SEQ ID NO:1 and its reaction substrate.

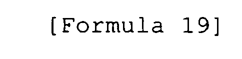

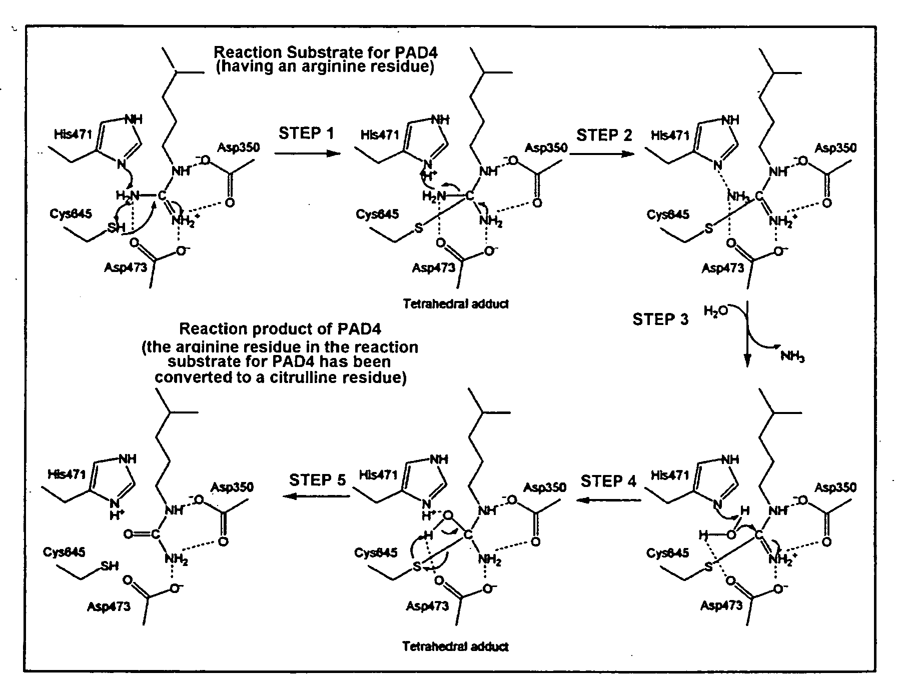

In the scheme, Asp350, His471, Asp473 and Cys645 represent an aspartic acid residue at position 350, a histidine residue at position 471, an aspartic acid residue at position 473 and a cysteine residue at position 645, respectively, in the amino acid sequence depicted in SEQ ID NO:1.
The substance capable of inhibiting any one of the steps 1 to 5 in the reaction mechanism between peptidylarginine deiminase 4 having the amino acid sequence depicted in SEQ ID NO: 1 and its substrate may be an arginine derivative. The arginine derivative may be such that each of the amino and guanidino groups in arginine has a substituent while the carboxyl group in arginine optionally has a substituent. Specifically, the arginine derivative is a compound represented by the general formula (I) or a salt thereof.
The substance capable of inhibiting any one of the steps 1 to 5 in the reaction mechanism between peptidylarginine deiminase 4 having the amino acid sequence depicted in SEQ ID NO:1 and its substrate can be examined utilizing all or part of the three-dimensional structural coordinates of peptidylarginine deiminase 4 or its protein mutants thereof. For example, a substance which can be recognized by peptidylarginine deiminase 4 having the amino acid sequence depicted in SEQ ID NO: 1 is examined (e.g., identified, searched, evaluated or designed) on a computer system utilizing all or part of the three-dimensional structural coordinates of Ca²⁺-free PAD4 deposited in the Protein Data Bank (accession code: 1WD8) or all or part of coordinates where the root mean square deviations thereof for bond length and bond angle are 0.019 angstrom and 1.894°, respectively; all or part of the three-dimensional structural coordinates of Ca²⁺-bound PAD4 (C645A) deposited in the Protein Data Bank (accession code: 1WD9) or all or part of coordinates where the root mean square deviations thereof for bond length and bond angle are 0.017 angstrom and 1.662°, respectively; or all or part of the three-dimensional structural coordinates of a PAD4(C645)-calcium ion-substrate (benzoyl-L-arginine-amide: BA) complex deposited in the Protein Data Bank (accession code: 1WDA) or all or part of coordinates where the root mean square deviations thereof for bond length and bond angle are 0.014 angstrom and 1.595°, respectively. Next, the substance is added with or substituted by an appropriate atom or atomic group at a proper position in the substance. In this manner, a substance capable of inhibiting any one of the steps 1 to 5 in the reaction mechanism between a substance recognized by peptidylarginine deiminase 4 having the amino acid sequence depicted in SEQ ID NO: 1 and its reaction substrate can be designed. The computer system to be used in the examination of the substance is not particularly limited, and any system may be used as long as a program for the examination of the substance can be run on it. Exemplary programs include DOCK (Science, 1992, 257, 1078), Gold4, Glide, FlexX (J. Mol. Biol., 1996, 261, 470), AutoDock (J. Comput. Chem., 1998, 19, 1639), ICM (J. Comput. Chem., 1994, 15, 488), and Ludi.
If it is desired to design a substance capable of inhibiting any one or all of the steps 1 to 5, it is preferred that the hydrogen atom on the group =NH₂(+) in arginine and/or the hydrogen atom on the group -NH₂ in arginine are/is substituted by an alkyl group (e.g., methyl group, ethyl group) and/or -NH be substituted by -CH²-.
The substance capable of inhibiting any one of the steps 1 to 5 in the reaction mechanism between peptidylarginine deiminase 4 having the amino acid sequence depicted in SEQ ID NO: 1 and its reaction substrate may be a naturally occurring or synthetic product, and it may be a polymeric or low-molecular compound.
The substance capable of inhibiting any one of the steps 1 to 5 in the reaction mechanism between peptidylarginine deiminase 4 having the amino acid sequence depicted in SEQ ID NO: 1 and its reaction substrate can be produced by any of the known procedures depending on the types of the substance.
Next, the interaction of the substance capable of inhibiting any one of the steps 1 to 5 in the reaction mechanism between peptidylarginine deiminase 4 having the amino acid sequence depicted in SEQ ID NO:1 and its reaction substrate exhibits with respect to peptidylarginine deiminase 4 (e.g., dissociation constant with respect to peptidylarginine deiminase 4), as well as the enzymatic activity of peptidylarginine deiminase 4 in the presence of the substance capable of inhibiting any one of the steps 1 to 5 in the reaction mechanism between peptidylarginine deiminase 4 having the amino acid sequence depicted in SEQ ID NO:1 and its reaction substrate may be determined. Peptidylarginine deiminase 4 can be prepared by any one of the known methods (e.g., the methods described in The Journal of Biological Chemistry, Vo.277, No.51, pp.49562-49568, 2002 and in the documents cited in the journal). The dissociation constant with respect to peptidylarginine deiminase 4 can be measured by performing a surface plasmon resonance experiment using BIACORE3000 (Pharamacia Biosensor AB). Briefly, peptidylarginine deiminase 4 is immobilized on the surface of a sensor chip, a substance to be tested is poured onto the sensor chip and, when the reaction system reaches an equilibrium, the dissociation constant is measured by the Schatchard plot analysis. The enzymatic activity of peptidylarginine deiminase 4 can be measured in accordance with the method described in Nakashima, K., Hagiwara, T., Ishigami, A., Nagata, S., Asaga, H., Kuramoto, M., Senshu, T. and Yamada, M. (1999) Molecular characterization of peptidylarginine deiminase in HL-60 cells induced by retinoic acid and 1a,25-dihydroxyvitamin D3. J. Biol. Chem., 274, 27786-27792. A substance capable of decreasing the enzymatic activity of peptidylarginine deiminase 4 can be used as a peptidylarginine deiminase 4 inhibitor.
The peptidylarginine deiminase 4 inhibitor of the present invention may be administered to a human or other animals in the form of a pharmaceutical preparation or it may be used as a reagent for experimental purposes. The peptidylarginine deiminase 4 inhibitor of the present invention may be used singly or in combination with other therapeutic agents (e.g., other prophylactic/therapeutic agents for rheumatoid arthritis).
When the peptidylarginine deiminase 4 inhibitor of the present invention is administered to a human, the inhibitor can be administered orally at about 0.1 to 9000 mg/kg body weight per day, preferably about 1 to 900 mg/kg body weight per day, in terms of the amount of the active ingredient, either as a single dose or in divided portions. However, the dose or the frequency of administration may vary as required, depending on the conditions or age of the patient, route of administration or the like.
The peptidylarginine deiminase 4 inhibitor of the present invention may be administered orally in the form of such preparations as tablet, capsule, granule, powder or syrup, or it may be administered parenterally in the form of such preparations as an injectable solution or suppository through intraperitoneal or intravenous injection. The content of the active ingredient in the preparation may vary within the range from 1 to 90% by weight. For example, when administered in the form of such preparations as tablet, capsule, granule or powder, the active ingredient is preferably contained in the preparation at a concentration of 5 to 80% by weight; when administered in the form of a liquid preparation such as a syrup, the active ingredient is preferably contained in the preparation at a concentration of 1 to 30% by weight; and when administered parenterally in the form of an injectable solution, the active ingredient is preferably contained in the solution at a concentration of 1 to 10% by weight.
The peptidylarginine deiminase 4 inhibitor of the present invention can be formulated into a pharmaceutical preparation in a conventional manner using pharmaceutical additives such as: excipients (e.g., saccharides including lactose, saccharose, glucose and mannitol; starches including potato, wheat and corn starches; inorganic substances including calcium carbonate, calcium sulfate and sodium hydrogen-carbonate; crystalline cellulose); binders (e.g., starch gel, gum arabic, gelatin, sodium alginate, methylcellulose, ethylcellulose, polyvinyl pyrrolidone, polyvinyl alcohol, hydroxylpropylcellulose, carmelose); lubricants (e.g., magnesium stearate, talc, hydrogenated vegetable oils, macrogol, silicone oil); disintegrants (e.g., starch, agar, gelatin powder, crystalline cellulose, CMC·Na, CMC·Ca, calcium carbonate, sodium hydrogen-carbonate, sodium alginate); flavoring agents (e.g., lactose, saccharose, glucose, mannitol, aromatic essential oils); solvents (e.g., water for injection, sterile purified water, sesame oil, soybean oil, corn oil, olive oil, cottonseed oil); stabilizers (e.g., inert gases including nitrogen and carbon dioxide; chelating agents including EDTA and thioglycolic acid; reducing agents including sodium hydrogen-sulfite, sodium thiosulfate, L-ascorbic acid and rongalit); preservatives (e.g., paraoxybenzoic acid ester, chlorobutanol, benzyl alcohol, phenol, benzalkonium chloride); surfactants (e.g., hydrogenated castor oil, polysorbate 80, polysorbate 20); buffering agents (e.g., sodium citrate, acetate or phosphate, boric acid); and diluents.
The peptidylarginine deiminase 4 inhibitor of the present invention can be used for the prevention and/or treatment of diseases associated with peptidylarginine deiminases. Diseases known to be associated with peptidylarginine deiminases include rheumatoid arthritis, psoriasis and multiple sclerosis and the peptidylarginine deiminase 4 inhibitor of the present invention is effective for the prevention and/or treatment of rheumatoid arthritis, multiple sclerosis and the like. The peptidylarginine deiminase 4 inhibitor of the present can also be used in the study of peptidylarginine deiminase 4.
The specification includes all or part of the contents as described in the specification and/or drawings of Japanese Patent Application No. 2004-28467, which is a priority document of the present application.

### Effect of the Invention

According to the present invention, a peptidylarginine deiminase 4 inhibitor is provided. The inhibitor can be used for the prevention and/or treatment of diseases associated with peptidylarginine deiminase (e.g., rheumatoid arthritis and multiple sclerosis).

### Brief Description of Drawings

Fig. 1 shows the schematic illustration of the reaction mechanism for deimination of PAD4 as proposed by the present inventors.
Fig. 2 shows the HPLC charts of final purified products produced in the Production Example, in which the reference number 1 represents a peak of Bz-Arg, 2 for a peak of Bz-Arg (mono-methyl), 3 for a peak of Bz-ADMA, and 4 for a peak of Bz-SDMA.
Fig. 3 shows the results of an inhibition reaction on the PAD4 digestion of the Bz-Arg derivatives produced in the Production Example (as determined 40 minutes after the reaction was initiated).
Fig. 4 shows the results of an inhibition reaction on the PAD4 digestion of the Bz-Arg derivatives produced in the Production Example (as determined 60 minutes after the reaction was initiated).

### Best Mode for Carrying out the Invention

Hereinbelow, the present invention will be described in great detail with reference to the following examples. Note that the examples are for illustrative purposes only and the scope of the invention is not limited to these examples.

### Examples

### [Production Example] Synthesis of Bz-Arg derivatives

Each of Arg derivatives (Arg: Nacalai Tesque Inc., Kyoto, Japan; citrulline: Sigma, St louis, USA; N^{G}-monomethyl-L-arginine: Wako Pure Chemical Industries, Ltd., Osaka, Japan; ADMA (N^{G},N^{G}-dimethyl-L-argnine): ALEXIS Biochemicals, Lausen, Switzerland; and SDMA (N^{G},N^{G'}-dimethyl-L-argnine): ALEXIS Biochemicals, Lausen, Switzerland) (10 µmol) was dissolved in 0.1 M NaHCO₃ (200 µl), and Bz₂O(10 µmol)/DMF(200 µl) was added to the solution. After stirring, the mixture was allowed to stand at room temperature for 1 hour. The reaction solution was diluted with water (200 µl), and then washed with ethyl acetate (500 µl) three times. The resulting aqueous solution was added with 6 M HCl (100 µl) and then washed with ethyl acetate (500 µl) four times. The resulting reaction solution was subjected to reverse-phase HPLV to purify the desired Bz-Arg derivative (1: Bz-Arg, 2: Bz-Arg (mono-methyl), 3: Bz-ADMA, 4: Bz-SDMA). After the purification, all of the Bz-Arg derivatives were obtained at yields of around 40%.
Conditions for HPLC
Waters M600 multi-solvent delivery system
UV: 220 nm
Column: Develosil ODS-UG-5 (4.6 x 150 mm)
Temp.: 30°C
Solvent: Starting from 5% acetonitrile in a 0.05% aqueous TFA solution, the concentration of acetonitrile was increased at a rate of 1%/min.
The HPLC charts of the final purified products are shown in Fig. 2, wherein the reference number 1 represents a peak of Bz-Arg, 2 for a peak of Bz-Arg (mono-methyl), 3 for a peak of Bz-ADMA, and 4 for a peak of Bz-SDMA.
The individual compounds were identified by MALDI-TOF MS (mass spectrometry).
Apparatus: Applied Biosystems Voyager System 6178

**[Table 1]**

| Atoms | Accurate mass number | | |
|---|---|---|---|
| C | 12 | | |
| H | 1.00783 | | |
| N | 14.0031 | | |
| O | 15.9949 | | |

| | | MALDI-TOF Mass | |
|---|---|---|---|
| | Accurate mass number (M) | Calculated M+H | Found M+H |
| Bz-Arg | -278.1 | 279.1 | 279.5 |
| Bz-Arg (mono-methyl) | 292.2 | 293.2 | 293.6 |
| Bz-ADMA | 306.2 | 307.2 | 307.6 |
| Bz-SDMA | 306.2 | 307.2 | 307.6 |
| Bz-citrulline | 279.1 | 280.1 | 280.3 |

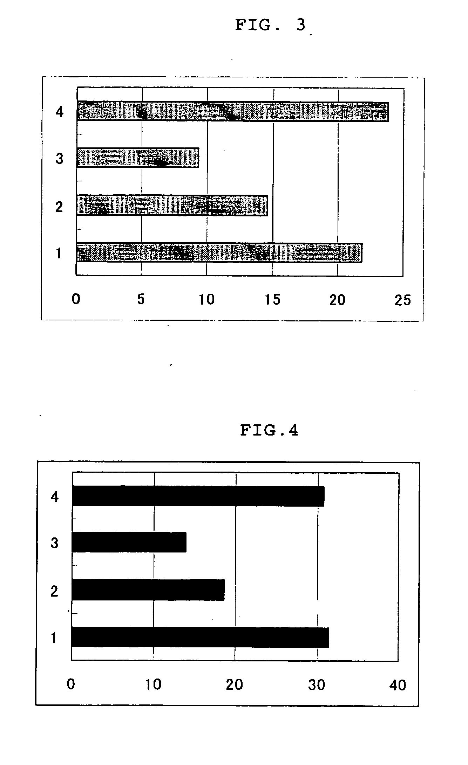

[Test Example] Inhibition reaction of Bz-Arg derivatives on PAD4 digestion
A buffer solution B (0.1 M Tris/HC1, 10 mM CaCl₂, 2 mM DTT, pH 7.6, 125 µl), Bz-Arg (0.1 M Tris/HCl, 10 mM CaCl₂, pH 7.6, 25 µl (a solution prepared in a concentration of 1 nmol/µl)) and PAD4 (1 µl) were mixed together under ice-cooling to give a Bz-Arg solution. PAD4 was prepared in accordance with the methods described in The Journal of Biological Chemistry, Vol.277, No.51, pp.49562-49568, 2002 and in the documents cited in the journal. Twenty µl each of Bz-Arg (mono-methyl), Bz-ADMA, Bz-SDMA and a buffer solution A (0.1 M Tris/HCl, 10 mM CaCl₂, pH 7. 6) (a solution prepared in a concentration of 1 nmol/µl) was mixed with the Bz-Arg solution (30 µl) and allowed to react at 37°C for 40 or 60 minutes. The reaction was quenched with 1 M HCl (50 µl) and then subjected to reverse-phase HPLC to separate the reaction mixture. As a result, Bz-ADMA was found to show the most potent inhibitory effect, followed by Bz-Arg (mono-methyl). Bz-SDMA showed no inhibitory effect at the concentration employed in the test. The results as determined 40 minutes and 60 minutes after the start of reaction are shown in Fig. 3 and Fig. 4, respectively. In Figs. 3 and 4, the reference number 1 represents the result with no inhibitor, 2 for the result with Bz-Arg (mono-methyl), 3 for the results with Bz-ADMA, and 4 for the result with Bz-SDMA; the vertical axis indicates the sample number and the horizontal axis indicates the yield of the deimination reaction (i.e., yield of the Bz-citrulline produced).
All publications, patents and patent applications cited herein are incorporated herein by reference in their entirety.

### Industrial Applicability

According to the present invention, a peptidylarginine deiminase 4 inhibitor is provided. The inhibitor can be used for the prevention and/or treatment of diseases associated with peptidylarginine deiminases (e.g., rheumatoid arthritis and multiple sclerosis).

### Free Text of Sequence Listing

SEQ ID NO:1 shows the amino acid sequence of human peptidylarginine deiminase 4.

## Claims

1. A compound represented by the general formula (I) or a salt thereof: 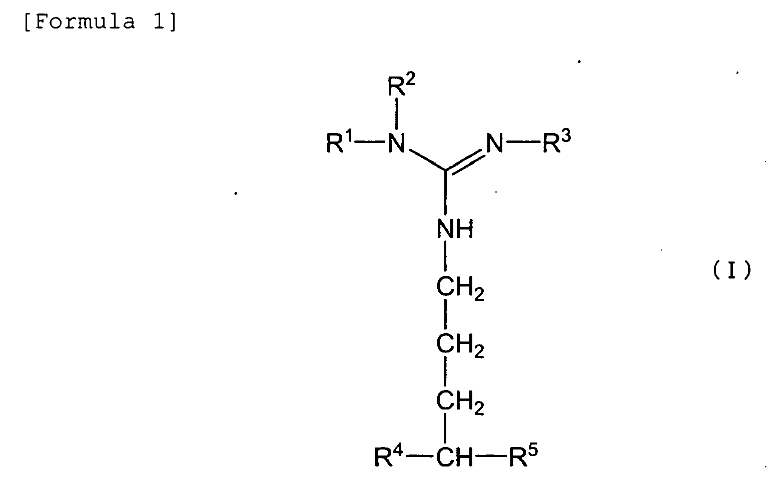 wherein R¹, R² and R³ each independently represent a hydrogen atom or an alkyl group having 1 to 3 carbon atoms, provided that at least one of R¹, R² and R³ does not represent a hydrogen atom; R⁴ represents an amino group which has a substituent; and R⁵ represents a carboxyl group which may have a substituent.

2. The compound or salt thereof according to Claim 1, wherein R⁴ represents the following formula: 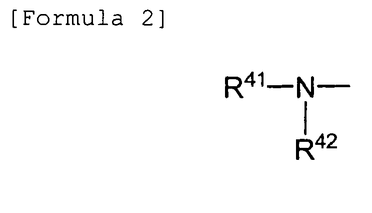 wherein R⁴¹ represents a group represented by R⁴⁰¹CO- where R⁴⁰¹ represents a hydrogen atom, a hydrocarbon group which may have a substituent or a heterocyclic group which may have a substituent, a group represented by R⁴⁰²S (O)ₘ- where R⁴⁰² represents a hydrogen atom, a hydrocarbon group which may have a substituent or a heterocyclic group which may have a substituent, and m is an integer of 1 or 2, a group represented by
R⁴⁰⁵N (R⁴⁰⁶) -CHR⁴⁰⁴-CO- [NH-CHR⁴⁰³-CO]ₙ- where R⁴⁰³, R⁴⁰⁴, R⁴⁰⁵ and R⁴⁰⁶ each independently represent a hydrogen atom, a hydrocarbon group which may have a substituent or a heterocyclic group which may have a substituent, and n is an integer of 1 to 50, or a peptidyl group which may have a substituent; and R⁴² represents a hydrogen atom or an alkyl group having 1 to 3 carbon atoms.

3. The compound or salt thereof according to Claim 2, wherein R⁴¹ represents a benzoyl group which may have a substituent, a benzoylpeptidyl group which may have a substituent, a dansyl group which may have a substituent or a dansylpeptidyl group which may have a substituent; and R⁴² represents a hydrogen atom.

4. The compound or salt thereof according to any one of Claims 1 to 3, wherein R¹, R² and R³ each independently represent a hydrogen atom or a methyl group, provided that at least one of R¹, R² and R³ represents a methyl group.

5. The compound or salt thereof according to Claim 4, which is a compound represented by the formula (Ia), (Ib) or (Ic) or a salt thereof.

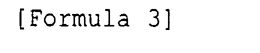

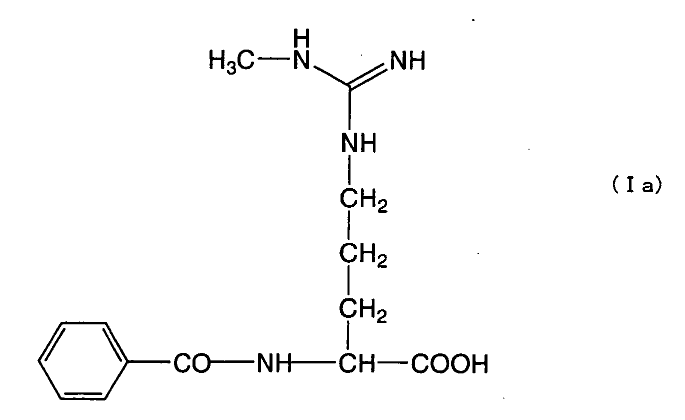

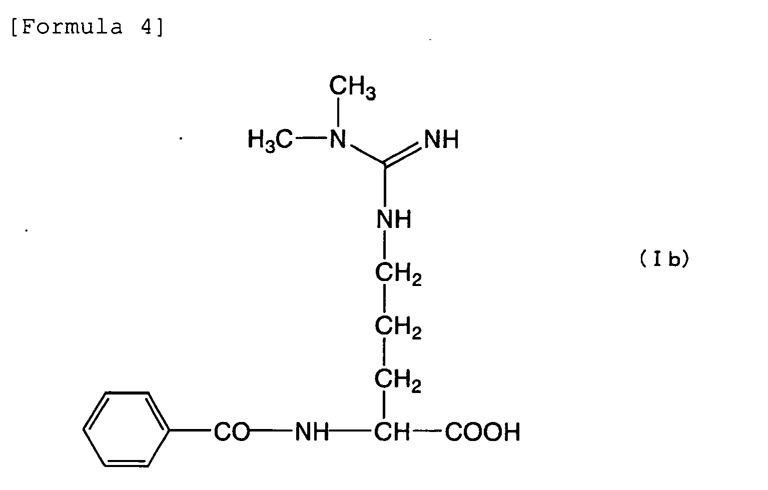

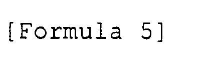

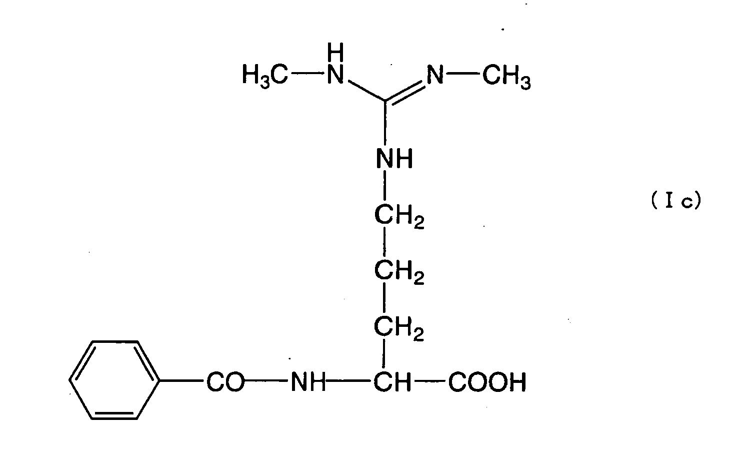

6. A peptidylarginine deiminase 4 inhibitor comprising, as the active ingredient, a substance capable of inhibiting any one of the steps 1 to 5 in the reaction mechanism as shown in the following scheme between peptidylarginine deiminase 4 having the amino acid sequence depicted in SEQ ID NO:1 and its reaction substrate. 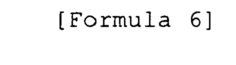 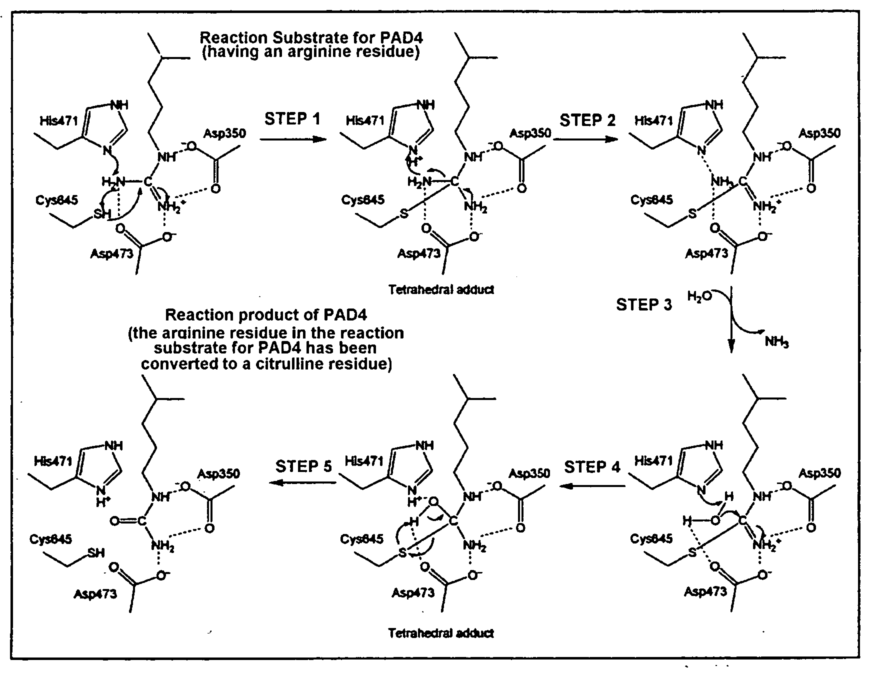 In the scheme, Asp350, His471, Asp473 and Cys645 represent an aspartic acid residue at position 350, a histidine residue at position 471, an aspartic acid residue at position 473 and a cysteine residue at position 645, respectively, in the amino acid sequence depicted in SEQ ID NO:1.

7. The peptidylarginine deiminase 4 inhibitor according to Claim 6, wherein the substance capable of inhibiting any one of the steps 1 to 5 in the reaction mechanism between peptidylarginine deiminase 4 having the amino acid sequence depicted in SEQ ID NO:1 and its reaction substrate is an arginine derivative.

8. A peptidylarginine deiminase 4 inhibitor comprising, as the active ingredient an arginine derivative having a substituent on each of the amino and guanidino groups in arginine and optionally having a substituent on the carboxyl group in arginine.

9. The peptidylarginine deiminase 4 inhibitor according to Claim 7 or 8, wherein the arginine derivative is a compound or a salt thereof as recited in any one of Claims 1 to 5.

10. The peptidylarginine deiminase 4 inhibitor according to any one of Claims 6 to 9, which is used for the prevention and/or treatment of diseases associated with peptidylarginine deiminases.

11. The peptidylarginine deiminase 4 inhibitor according to Claim 10, wherein the diseases associated with peptidylarginine deiminase are selected from the group consisting of rheumatoid arthritis, psoriasis, and multiple sclerosis.
